# EUROPÄISCHE PATENTSCHRIFT

(11) **EP 2 682 161 B1**
(45) Veröffentlichungstag und Bekanntmachung des Hinweises auf die Patenterteilung: **06.07.2016**
(21) Anmeldenummer: 13166977.2
(22) Anmeldetag: 08.05.2013
(51) Int. Cl.: A61Q 5/12, A61Q 5/02, A61Q 19/10, A61K 8/44

(54) **Flüssiges Reinigungs- und Pflegemittel**
Liquid cleaning and care agent
Agent de nettoyage et d'entretien liquide

(30) Priorität: 16.05.2012 DE 102012104281
(43) Veröffentlichungstag der Anmeldung: 08.01.2014
(73) Patentinhaber: Fitzner, Sabine, 51147 Köln (DE)
(72) Erfinder: Fitzner, Sabine, 51147 Köln (DE)
(74) Vertreter: Fritz, Edmund Lothar

(56) Entgegenhaltungen:
- WO-A1-2010/069500
- DE-A1- 19 723 763

## Beschreibung

Die vorliegende Erfindung betrifft ein flüssiges Reinigungs- und Pflegemittel für die Haar- und Körperreinigung mit guter Hautverträglichkeit, enthaltend wenigstens ein pH-sensitives Aminosäuretensid, Wasser, sowie mindestens einen pH-Wert-Regulator.

Reinigungsmittel, versetzt mit oberflächenaktiven Stoffen, werden seit Jahrtausenden in Form von Seifenlösungen verwendet. Bis zum heutigem Tag haben sich daraus in der Kosmetik immer komplexere und hautverträglichere Formulierungen mit ionischen und nichtionischen Tensiden, angereichert mit Wirk-, Pflege- und Marketingsubstanzen, optimiert für den jeweiligen Einsatzzweck, entwickelt. Bei den Produkten aus dem Bereich Putz- und Reinigungsmittel finden sich dagegen häufig Formulierungen aus wenigen Bestandteilen, gegebenenfalls unter Zugabe von (polymeren) Wirkstoffen zur Oberflächenmodifikation.

Die hier vorzustellende Erfindung kann übergreifend in Reinigungs- und/oder Pflegeprodukten eingesetzt werden, aufgrund der Marktbedeutung wird der Effekt detailliert für die kosmetische Anwendung dargestellt.

Marktgängige kosmetische Formulierungen zur Haar- und Körperreinigung basieren zu einem hohen Prozentsatz auf Natrium Laureth Sulfat und Cocamidopropylbetain mit Aktivgehalten der Tenside in den Endverbraucherprodukten (Duschbäder, Shampoos, Flüssigseifen, Schaumbäder etc.) zwischen ca. 8% bis 16%. Die Produkte zeichnen sich durch eine marktübliche Sensorik, Schaumentwicklung und Fließeigenschaften/Viskosität aus. Die Viskosität muß so hoch sein, dass das Produkt bei der Anwendung (Aufgiessen auf die Hand) nicht durch die Finger wegläuft bzw. bei der Körperreinigung nicht von der Haut abläuft. Dies kann durch die marktübliche Verwendung von Natriumchlorid-verdickbaren Tensidsystemen mit entsprechender WAS (waschaktiver Substanz) erreicht werden. Bei kostengünstigeren Produkten (Formulierungen mit geringer WAS Konzentration) werden (Polymer)Verdicker (Xanthan Gum oder modifizierte Acrylate) oder hohe Natriumchloridkonzentrationen zur Verdickung eingesetzt. Damit sind hohe Viskositäten zur marktgerechten Handhabung erzielbar, die Sensorik dieser Produkte ist jedoch schleimig, fädenziehend mit schlechten Löseeigenschaften aufgrund der gelartigen Struktur. Der Endverbraucher erkennt diese Produkte als minderwertige Qualität, eine hohe Salzkonzentration kann zusätzlich zu Problemen mit der Augenschleimhautverträglichkeit führen.

Die gute Fettemulgierung des Basistensids Natrium Laureth Sulfat reinigt zwar gut und ist akzeptabel mild, führt aber bei häufiger Verwendung und/oder empfindlicher und trockener Haut zu Spannungsgefühlen der betroffenen Hautareale. Zusätzlich stört Natrium Laureth Sulfat bei diesen Hauttypen die Barrierefunktion durch die hohe Affinität der Laureth Sulfat Moleküle zur Hautoberfläche. Menschen mit diesen Problemen müssen daher häufig zusätzliche Hautpflegeprodukte verwenden, um diesen entfettenden Effekt auszugleichen. Speziell im gewerblichen Bereich (zum Beispiele Friseure oder Mitarbeiter in Gaststätten) gibt es dadurch Probleme bis hin zu Berufsunfähigkeiten durch Hautdermatosen an den Händen.

Der nachteilige Effekt der Hautentfettung und die Anstrengungen, dies auszugleichen, wird auch in der DE 10 2006 59 465 beschrieben.

Weiterhin stehen Kosmetikprodukte mit ethoxylierten Bestandteilen (Sogenannte "PEG-Verbindungen") in negativem Ansehen bei Produktvergleichen in Ökomagazinen und in der Naturkosmetik. Dies führte zur Nachfrage nach Formulierungen ohne ethoxylierte Inhaltsstoffe in der Kosmetik und nachfolgend zu einer Vielzahl von Rohstoffen auf Glycerinbasis (Beispiele: Glyceryl Stearate Citrate (Axol C62 Evonik) oder Polyglyceryl-3 Polyricinoleate (Dermofeel® PR, Dr. Straetmans) und den entsprechenden Leave-on Marktprodukten (Emulsionen und Cremes) ohne "PEGs".

Eine entsprechende Entwicklung hat es trotz der Markteinführung von neuen nichtethoxilierten Tensiden (Alkylpolyglycoside wie Plantacare®, Cognis oder Natrium Acylglutamat, Cognis oder Ajinomoto) im rinse-off Bereich der Kosmetik nicht gegeben. Die neuen Alternativrohstoffe waren wie die etablierten nichtethoxilierten Tenside Cocamidopropylbetain, Cocoamphoacetate oder Sulfosuccinate in der Kosten-Nutzen Betrachtung nicht gleichwertig zu Natrium Laureth Sulfat. Im allgemeinem müssen erhöhte WAS-Konzentrationen zur marktüblichen Schaumentwicklung eingesetzt werden sowie ethoxylierte oder polymere Verdicker zur Viskositätserhöhung.

In Naturkosmetikformulierungen setzen Hersteller deshalb teilweise auch das stärker hautreizende Tensid Natrium Lauryl Sulfat oder dessen Homologe ein, das nicht ethoxyliert ist. Natrium Lauryl Sulfat, Hauptanwendung heute als Waschmittelrohstoff, wird in der Kosmetik in Deutschland ansonsten kaum noch als Basistensid verwendet und ist Negativstandard im Hautverträglichkeitstest.

Ethoxylatfreie Tenside haben dabei auch den Vorteil der Ressourcenschonung, da der Reaktionschritt der Ethoxylierung in der Tensidherstellung und das Ethylenoxid selbst wegfällt.

Milde Formulierungen ohne Natrium Laureth Sulfat zeigen bislang folgende Nachteile: Sie sind vergleichsweise teuer, da die milden Tenside bei deutlich niedrigerem Aktivgehalt der Handelsform des Rohstoffes deutlich höher dosiert werden müssen, um den gleichen Tensidaktivgehalt wie Natrium Laureth Sulfat Rezepturen und ggfs. einen verdickbaren WAS-Gehalt zu erreichen.

### Beispiel zum Preisvergleich:

7,143 % 70 %iges Natrium Laureth Sulfat (Handelsform) zum Preisniveau 100 ergibt 5% Tensid Natrium Laureth Sulfat aktiv zum "Preis" von 100 mal 7,143% ergibt 714,3 Preiseinheiten;
12,5 % 40 %iges Cocamidopropylbetain (Handelsform) zum Preisniveau 80 ergibt 5% Tensid Cocamidopropylbetain aktiv zum "Preis" von 80 mal 12,5% ergibt 1000 Preiseinheiten;
15 % 30%iges Natrium Laurylsarkosinat (Handelsform) zum Preisniveau 150 ergibt 5% Tensid Natrium Laurylsarkosinat aktiv zum "Preis" von 150 mal 15% ergibt 2250 Preiseinheiten;

Es resultiert also für den gleichen WAS Gehalt eine deutlich bis mehr als doppelt so hohe Dosierung bei höherem Preisniveau der Handelsform zu schlussendlich den mehrfachen Rezepturkosten ohne Berücksichtigung der unterschiedlichen Rohstoffeigenschaften bzgl. Schaumentwicklung und Verdickung.

Die absoluten Preise variieren natürlich und sind auch vom Einkaufsvolumen der jeweiligen Unternehmen abhängig, grundsätzlich ist aber Natrium Laureth Sulfat (70% WAS) aufgrund der hohen produzierten und verbrauchten Menge deutlich preiswerter als milde Tenside wie Natrium Lauroylsarkosinat (30% WAS) und Cocamidopropylbetain (40 % WAS).

Dies verdeutlicht den hohen wirtschaftlichen Nachteil milder Formulierungen, der noch durch zusätzliche Kosten für Verdicker zur Herstellung einer marktgerechten Viskosität verstärkt wird und unterstreicht damit die große Bedeutung der vorliegenden Erfindung zur Herstellung milder, marktgerechter Produkte.

Formulierungen mit milden Tensiden können in der Regel nicht oder wie oben erläutert nur bei hohen WAS-Gehalten wie Natrium Laureth Sulfat basierte Rezepturen über Elektrolytzugabe (Natriumchlorid etc.) in eine marktgängige Viskosität verdickt werden, ansonsten nur durch Zugabe externer Verdicker. Beispiele für externe Verdicker sind PEG-120 Dioleate, PEG-150 Distearate oder PEG-200 Hydrogenated Palmitate; Xanthan Gum oder modifizierte Acrylate.

In der DE 197 23 763 A1 ist ein Haarwaschmittel beschrieben, welches ein Aminosäuretensid wie beispielsweise Natriumlauroylglutamat im Gemisch mit weiteren Tensiden wie zum Beispiel Cocoamidopropylbetain sowie weiteren Bestandteilen enthält, darunter auch Citronensäure als organische Säure. Es wird angegeben, dass der pH-Wert des Shampoos innerhalb eines weiten Bereichs liegen kann, wobei von einem üblichen Bereich zwischen etwa 5 und 8,5 die Rede ist. Dies umfasst auch pH-Werte, die in der Fachwelt nicht als pH-hautneutral angesehen werden. Die Viskosität des Shampoos soll ebenfalls in einem üblichen Bereich liegen, der mit zwischen etwa 100 und etwa 10 000 mPa.s bei 20 °C angegeben wird. In dieser Druckschrift wird weder empfohlen, den pH-Wert auf einen spezifischen engen Bereich einzustellen, noch finden sich Ausführungen dazu, dass der pH-Wert des Tensidgemischs dessen Viskosität beeinflusst. Es wird auch nicht erwähnt, dass die Citronensäure dazu dient, den pH-Wert einzustellen, um die Viskosität des Gemischs zu beeinflussen.

In der US 5,866,110 A werden Reinigungsmittelzusammensetzungen beschrieben, die Natriumlauroylsarkosinat im Gemisch mit Cocoamidopropylbetain, Wasser, Citronensäure und weitere Bestandteile enthalten. Die Vorgehensweise bei der Herstellung ist jedoch so, dass nach Zugabe des Cocamidopropylbetains, das ein amphoteres Tensid und kein pH-sensitives Aminosäuretensid ist, zunächst der pH-Wert durch Zugabe von Citronensäure auf 5,6 bis 5,9 gesenkt wird. Dies geschieht, um den isoelektrischen Bereich des amphoteren Tensids einzustellen. Dabei besteht kein Zusammenhang zwischen dem pH-Wert und der Viskosität des Gemischs. Erst danach wird das Aminosäuretensid Natriumlauroylsarkosinat zugegeben. Das Gemisch wird dann erhitzt und der pH-Wert auf 6,1 bis 6,3 eingestellt. Es besteht wiederum kein Zusammenhang mit der Viskosität des Gemischs. Anschließend wird dann die Erhöhung der Viskosität durch die Zugabe eines Verdickungsmittels, nämlich Natriumsulfat vorgenommen. Es wird somit in dieser Druckschrift die Zugabe eines Verdickers für erforderlich gehalten, um eine erhöhte Viskosität der Tensidmischung zu erreichen und es wird nicht erkannt, dass sich die Viskosität des Tensidgemischs aufgrund von Eigenschaften des Aminosäuretensids durch Veränderung des pH-Werts beeinflussen lässt. Auch in anderen Beispielen werden in dieser Druckschrift dem Tensidgemisch Verdicker in Form von C12/14 Fettalkoholen zugegeben.

In der WO 2010/069500 A1 wird ein flüssiges Reinigungs- und Pflegemittel der eingangs genannten Art beschrieben, welches neben einem Aminosäuretensid weitere waschaktive Substanzen wie beispielsweise amphotere Tenside enthält. Als Aminosäuretensid wird in den Formulierungen auch Natriumlauroylsarkosinat eingesetzt, wobei die Aminosäuretenside insgesamt in vergleichsweise großen Mengen von 9 % oder 10 % verwendet werden und der pH-Wert bei Verwendung von Natriumlauroylsarkosinat auf etwa 5 eingestellt wird. Bei diesem pH-Wert zeigt das Gemisch keine wesentliche Viskositätserhöhung. Daher wird in der genannten Schrift auch dem Tensidgemisch noch ein Verdicker wie PED-90 Glycerylisostearat oder PEG-160 Sorbitantriisostearat zugesetzt. Die Aminosäuretenside sind vergleichsweise teuer, so dass deren Verwendung in großen Anteilen in dem Reinigungsmittel kostspielig ist. Die zusätzliche Beimischung von Verdickern verteuert das Reinigungsmittel weiter und steht einem Einsatz in einem industriellen Produkt, das sich am Markt behaupten kann, entgegen. Die Verfasser der genannten Schrift aus dem Stand der Technik haben offensichtlich den Zusammenhang zwischen dem pH-Wert der Gemisch und deren Viskosität nicht erkannt, weshalb die Zugabe von Verdickern für notwendig erachtet wird, um dem Produkt die erforderliche dickflüssige Konsistenz zu geben.

Ausgehend von dem zuvor geschilderten Stand der Technik besteht die Aufgabe der vorliegenden Erfindung darin, ein flüssiges Reinigungsmittel zur Verfügung zu stellen, welches bei Verwendung von nicht ethoxylierten milden Tensiden eine gute Schaumentwicklung aufweist und gute sensorische und fließtechnische Eigenschaften hat, bei geringen, ökologisch und ökonomisch bevorzugten Tensidaktivgehalten, und welches vergleichsweise kostengünstig herstellbar ist.

Die Lösung dieser Aufgabe liefert ein erfindungsgemäßes Reinigungs- und Pflegemittel mit den Merkmalen des Anspruchs 1.

Das erfindungsgemäße Reinigungs- und Pflegemittel enthält wenigstens ein pH-sensitives Aminosäuretensid, Wasser, sowie mindestens einen pH-Wert-Regulator, der den pH-Wert des Gemischs auf einen spezifischen engen pH-Wert-Bereich von +/- 0,3 oder weniger bezogen auf einen pH-Wert einstellt, in dem das Gemisch ein absolutes Viskositätsmaximum zeigt oder eine relative Viskositätserhöhung von mindestens 100 % gegenüber einer Lösung dieses Aminosäuretensids in Wasser zeigt.

Im Rahmen der vorliegenden Erfindung wurde die Viskosität von Aminosäuretensiden sowie entsprechender Gemische aus diesen mit anderen Tensiden in Abhängigkeit vom pH-Wert gemessen. Wenn man die Ergebnisse dieser Messungen als Diagramm graphisch darstellt, lässt sich jeweils ein pH-Wert-Bereich ermitteln, in dem die Viskosität pH-abhängig ansteigt und nach Erreichen eines Maximums wieder abfällt. Dieser Bereich um das Viskositätsmaximum herum, in dem das Gemisch eine nennenswert erhöhte, das heißt messbar erhöhte, Viskosität zeigt, ist der "pH-Wert-Bereich" im vorgenannten Sinne.

Je nachdem, ob das Reinigungs- und Pflegemittel nur ein pH-sensitives Aminosäuretensid enthält oder eine Mischung aus zwei oder mehreren dieser Tenside oder eine Mischung mit anderen Tensiden, liegt bei den bisher untersuchten Mischungen das Viskositätsmaximum im pH-Wert-Bereich von etwa 4,0 bis 7,5. Die einzelnen bislang untersuchten pH-sensitiven Tenside, beispielsweise Aminosäuretenside, haben meist Viskositätsmaxima im pH-Bereich von etwa 4,5 bis 5,7; die binären Mischungen meist im pH-Bereich von etwa 4,2 bis 7,0.

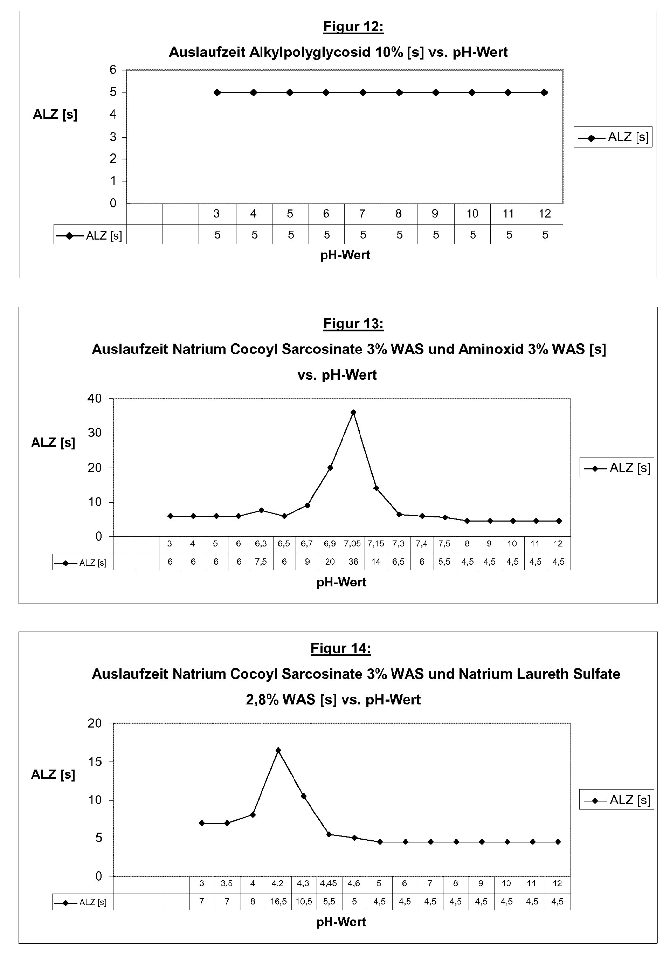

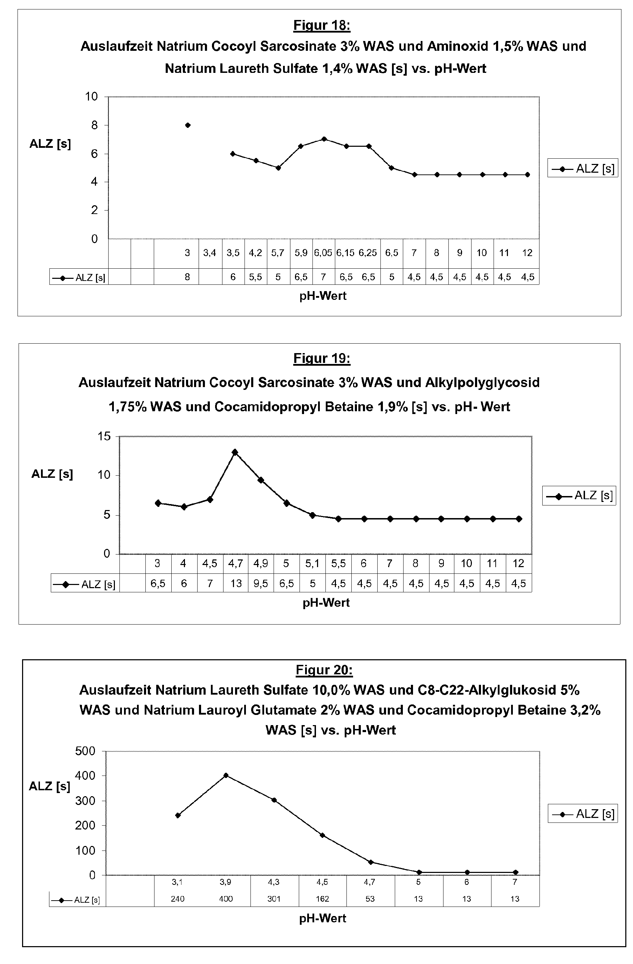

Die Lage des Viskositätsmaximums und die Breite des Bereiches erhöhter Viskosität wird dabei in ternären und komplexeren Tensidmischungen sowohl durch das Verhältnis der Tenside zueinander beeinflusst als auch durch den Tensidgehalt der Lösung als auch durch die Lage der pH-Viskositätsmaxima der jeweiligen binären Mischungen zueinander. Je weiter die jeweiligen binären Viskositätsmaxima einer ternären oder höheren Tensidmischung gleichen Tensidgehaltes voneinander entfernt sind, desto weniger synergistisch ist die maximale Viskositätserhöhung und desto breiter der Bereich der Viskositätserhöhung. Dies kann in Figur 18 am Beispiel des Viskositätsverlaufes von Natrium Cocoyl Sarcosinate 3% WAS und Lauramine Oxide 1,5% WAS und Natrium Laureth Sulfate 1,4% WAS im Vergleich zu den Figuren 13 und 14 der binären Mischungen dokumentiert werden.

Der Begriff "pH-sensitives Aminosäuretensid" bedeutet im Sinne der vorliegenden Erfindung, dass das Aminosäuretensid in wässriger Lösung in einem spezifischen, meist vergleichsweise engen pH-Bereich eine nennenswerte Viskositätserhöhung zeigt. Wichtig für das Verständnis der Erfindung ist, dass die erfindungsgemäße Viskositätserhöhung bei einem bestimmten Aminosäuretensid oder bei einer konkreten binären Mischung mit einem weiteren Tensid oder einer konkreten ternären Mischung mit zwei weiteren Tensiden immer in einem vergleichsweise engen pH-Wert-Bereich auftritt, dieser pH-Wert-Bereich aber bei unterschiedlichen Aminosäuretensiden oder Tensidmischungen der genannten Art durchaus unterschiedlich sein kann.

Wenn beispielsweise das pH-sensitive Aminosäuretensid Natriumcocoylsarkosinat ist, wird mittels des pH-Wert-Regulators der pH-Wert auf einen Bereich von etwa 4,65 bis 5,25 (entspricht 4,95 +/- 0,3) eingestellt.

Wenn beispielsweise das pH-sensitive Aminosäuretensid Natriumlauroylsarkosinat ist, wird mittels des pH-Wert-Regulators der pH-Wert auf einen Bereich von etwa 4,56 bis 5,16 (entspricht 4,86 +/- 0,3) eingestellt.

Wenn beispielsweise das pH-sensitive Aminosäuretensid Natriumcocoylglutamat ist, wird mittels des pH-Wert-Regulators der pH-Wert auf einen Bereich von etwa 4,3 bis 4,6 (entspricht 4,45 +/- 0,15) eingestellt.

Wenn beispielsweise das pH-sensitive Aminosäuretensid Natriumcocoylalaninat ist, wird mittels des pH-Wert-Regulators der pH-Wert auf einen Bereich von etwa 5,4 bis 6,0 (entspricht 5,7 +/- 0,3) eingestellt.

Wenn das Reinigungsmittel beispielsweise ein binäres Tensidgemisch aus Natriumcocoylsarkosinat (3% waschaktive Substanz (WAS) als pH-sensitives Aminosäuretensid sowie Cocamidopropylbetain (3,8% WAS) enthält, wird mittels des pH-Wert-Regulators der pH-Wert auf einen Bereich von etwa 4,5 bis 5,0 (entspricht 4,75 +/- 0,25) eingestellt.

Wenn beispielsweise das Reinigungsmittel ein binäres Tensidgemisch aus Natriumcocoylsarkosinat (3% WAS) als pH-sensitives Aminosäuretensid sowie einem Aminoxid (3% WAS) enthält, wird mittels des pH-Wert-Regulators der pH-Wert auf einen Bereich von etwa 6,75 bis 7,35 (entspricht 7,05 +/- 0,3) eingestellt.

Wenn beispielsweise das Reinigungsmittel ein ternäres Tensidgemisch aus Natriumcocoylsarkosinat (3% WAS) als pH-sensitives Aminosäuretensid, Cocamidopropylbetain (1,9% WAS) sowie einem Aminoxid (1,5% WAS)" insbesondere Lauraminoxid, enthält, wird mittels des pH-Wert-Regulators der pH-Wert auf einen Bereich von etwa 5,9 bis 6,5 (entspricht 6,2 +/- 0,3) eingestellt.

Einige pH-Werte, bei denen gemäß der vorliegenden Erfindung Viskositätsmaxima auftreten, sind für einige Einzelrohstoffe, binäre, ternäre und komplexere Gemische exemplarisch in der nachfolgenden Tabelle zusammengestellt:

### Übersicht der pH-Werte der Viskositätsmaxima und der Bereiche erhöhter Viskosität:

**Einzelrohstoffe:**

| | pH-Wert |
|---|---|
| Natrium Cocoyl Sarkosinat 10% WAS | 4,95+/-0,1 |
| Natrium Lauroyl Sarkosinat 9% WAS | 4,86+/-0,1 |
| Di Natrium Cocoyl Glutamate 10% WAS | 4,45 +/- 0,15 |
| Natrium Cocoyl Alaninate 10% WAS | 5,7+/-0,3 nicht |
| Natrium Cocoyl Glycinate 10% WAS | bestimmbar |

**Binäre Mischungen:**

| | |
|---|---|
| Natrium Cocoyl Sarcosinate 3% WAS und Cocamidopropyl Betaine 3,8% WAS | 4,75 +/- 0,25 |
| Natrium Cocoyl Sarcosinate 3% WAS und Lauramine Oxide 3% WAS | 7,05+/-0,3 |
| Natrium Cocoyl Sarcosinate 3% WAS und Natrium Laureth Sulfate 2,8% WAS | 4,2+/-0,2 |
| Natrium Cocoyl Sarcosinat 3% WAS und CocoGlucoside 3,5% WAS | 4,45 +/- 0,25 |
| Di Natrium Cocoyl Glutamate 3% WAS und Lauramine Oxide 3% WAS | 6,6+/-0,3 |
| Natrium Cocoyl Alaninate 3% WAS und Lauramine Oxide 3% WAS | 7,3+/-0,25 |
| Natrium Cocoyl Glycinate 3% WAS und Lauramine Oxide 3% WAS | 7,4+/-0,2 |

**Ternäre Systeme:**

| | |
|---|---|
| Natrium Cocoyl Sarcosinate 3% WAS und Cocamidopropyl Betaine 1,9% WAS und Lauramine Oxide 1,5% WAS | 6,2+/-0,3 |
| Natrium Cocoyl Sarcosinat 3% WAS und Cocamidopropyl Betaine 1,9% WAS und Natrium Laureth Sulfate 1,4% WAS | 4,8+/-0,3 |
| Cocamidopropyl Betaine 5,15% WAS und Natrium Laureth Sulfate 5,8% WAS und Natrium Lauroyl Sarcosinat 0,5% WAS | 4,0+/-0,7 |
| Natrium Cocoyl Sarcosinate 3% WAS und Lauramine Oxide 1,5% WAS und Natrium Laureth Sulfate 1,4% WAS | 6,05+/-0,2 |
| Natrium Cocoyl Sarcosinate 3% WAS und CocoGlucoside 1,75% WAS und Cocamidopropyl Betaine 1,9% WAS | 4,7+/-0,2 |

**Quaternäres System:**

| | |
|---|---|
| Natrium Laureth Sulfate 10,0% WAS und C8-C22-Alkylglukosid 5% WAS und Natriumlauroylglutamate 2% WAS und Cocamidopropyl Betaine 3,2% WAS | 3,9+/-0,8 |

Vorzugsweise enthält das erfindungsgemäße Reinigungs- und Pflegemittel neben dem pH-sensitiven Aminosäuretensid wenigstens ein weiteres nicht pH-sensitives anionisches Tensid und/oder wenigstens ein nichtionisches Tensid und/oder wenigstens ein kationisches und/oder ein amphoteres Tensid.

Vorzugsweise enthält das erfindungsgemäße Reinigungs- und Pflegemittel insgesamt nur von etwa 4 % bis etwa 16 % an waschaktiven Substanzen, insbesondere insgesamt etwa 4 % bis etwa 16 % der genannten Tenside, wobei es pH-sensitive Aminosäuretenside bevorzugt in einer Menge von etwa 1,8 Gew. % bis etwa 5 Gew. % enthält.

Besonders bevorzugt wird das pH-sensitive Aminosäuretensid ausgewählt aus der Gruppe umfassend Acylglutamate, Acylsarkosinate, Acylglycinate, Acylalaninate, insbesondere Dinatriumcocoylglutamat, Natriumcocoylsarkosinat, Natriumlauroylsarkosinat, Natriumcocoylalaninat, Natriumcocoylglycinat und Natriumlauroylglutamat.

Acylsarkosinate gemäß obiger Definition sind Verbindungen der allgemeinen Formel:

RCON(CH₃)CH₂COOM,

in der R = Alkyl oder Alkenyl mit 6 bis 30 C-Atomen ist, bevorzugt mit 8 bis 18 C-Atomen,
und in der M ein Wasserstoffatom (freie Säure) oder ein Metallkation ist, beispielsweise ein Alkaliion, bevorzugt Na oder K oder eine andere Base wie beispielsweise Ammoniak oder Triethanolamin.

Acylglutamate gemäß obiger Definition sind Verbindungen der allgemeinen Formel:

RCONHCH(CH₂CH₂COOM)COOM,

in der R = Alkyl oder Alkenyl mit 6 bis 30 C-Atomen ist, bevorzugt mit 8 bis 18 C-Atomen,
und in der M ein Wasserstoffatom (freie Säure) oder ein Metallkation ist, beispielsweise ein Alkaliion, bevorzugt Na oder K oder eine andere Base wie beispielsweise Ammoniak oder Triethanolamin.

Acylalaninate gemäß obiger Definition sind Verbindungen der allgemeinen Formel:

RCONHCH(CH₃)COOM,

in der R = Alkyl oder Alkenyl mit 6 bis 30 C-Atomen ist, bevorzugt mit 8 bis 18 C-Atomen,
und in der M ein Wasserstoffatom (freie Säure) oder ein Metallkation ist, beispielsweise ein Alkaliion, bevorzugt Na oder K oder eine andere Base wie beispielsweise Ammoniak oder Triethanolamin.

Acylglycinate gemäß obiger Definition sind Verbindungen der allgemeinen Formel:

RCONHCH₂COOM,

in der R = Alkyl oder Alkenyl mit 6 bis 30 C-Atomen ist, bevorzugt mit 8 bis 18 C-Atomen,
und in der M ein Wasserstoffatom (freie Säure) oder ein Metallkation ist, beispielsweise ein Alkaliion, bevorzugt Na oder K oder eine andere Base wie beispielsweise Ammoniak oder Triethanolamin.

Als pH-Regulator können grundsätzlich alle Säuren und Basen verwendet werden, die sich für die Verwendung in einem Reinigungs- und Pflegemittel eignen, welches in der Regel mit der Haut in Kontakt kommt. Besonders bevorzugt wird als pH-Wert-Regulator mindestens eine organische Säure verwendet, beispielsweise Milchsäure oder Ameisensäure. Letztere hat den zusätzlichen Vorteil, dass sie auch eine konservierende Eigenschaft aufweist. Als pH-Regulator kommen weiterhin andere sauer oder basisch wirkende Rohstoffe in Betracht wie beispielsweise nicht neutralisierte Acylglutaminsäuren.

Das amphotere Tensid oder die amphoteren Tenside sind in dem erfindungsgemäßen Reinigungs- und Pflegemittel vorzugsweise in einer Menge von etwa 3 Gew.% bis etwa 8 Gew.% enthalten.

Das weitere nicht pH-sensitive anionische Tensid oder weitere anionische Tenside sind bevorzugt in einer Menge von bis zu etwa 5 Gew.% enthalten.

Nicht ionisches Tensid oder Tenside sind bevorzugt in einer Menge von bis zu etwa 5 Gew.% enthalten und/oder kationisches Tensid oder Tenside in einer Menge von bis zu etwa 3 Gew.%.

Das erfindungsgemäße Reinigungs- und Pflegemittel enthält vorzugsweise das pH-sensitive Aminosäuretensid in einer geringeren Menge als das amphotere Tensid und/oder die weiteren Tenside, beispielsweise in einem Gewichtsverhältnis pH-sensitives Aminosäuretensid zu amphoterem Tensid von 1: 1,5 oder größer.

Gegenstand der vorliegenden Erfindung ist weiterhin ein Reinigungs- und Pflegemittel, welches wenigstens ein pH-sensitives Aminosäuretensid gegebenenfalls im Gemisch mit weiteren Tensiden enthält und bei dem der pH-Wert des Gemischs mittels des pH-Wert-Regulators auf einen spezifischen engen pH-Wert-Bereich von +/- 0,3 oder weniger eingestellt ist, bezogen auf einen pH-Wert, in dem das Gemisch eine relative Viskositätserhöhung von mindestens 300 %, bevorzugt von mindestens 500 % gegenüber einem Gemisch gleicher Substanzen in Wasser zeigt, welches das oder die Aminosäuretenside nicht enthält. Es versteht sich, dass in der vorstehenden Definition einer Variante der Erfindung die durch Zugabe des pH-Wert-Regulators erzielte Viskositätserhöhung nur durch die Veränderung des pH-Werts und ohne Zugabe jeglicher Mittel erfolgt, die das Gemisch verdicken. Die Viskositätserhöhung kann entweder in Auslaufzeiten gemessen werden oder aber in mPas, wie in den nachfolgenden Beispielen und Vergleichsbeispielen der vorliegenden Anmeldung angegeben.

Gegenstand der vorliegenden Erfindung ist weiterhin ein Verfahren zur Herstellung eines solchen Reinigungs- und Pflegemittels mit den Merkmalen des Anspruchs 13. Danach ist vorgesehen, dass das Herstellungsverfahren mindestens einen Verfahrensschritt umfasst, in dem man einem Gemisch aus wenigstens einem pH-sensitiven Aminosäuretensid sowie gegebenenfalls weiteren nicht pH-sensitiven Tensiden und Wasser zur Einstellung der Viskosität mindestens einen pH-Wert-Regulator in einer solchen Menge zugibt, dass der pH-Wert des Gemischs auf einen spezifischen engen pH-Wert-Bereich von +/- 0,3 oder weniger bezogen auf einen pH-Wert eingestellt wird, in dem das Gemisch ein absolutes Viskositätsmaximum zeigt oder eine relative Viskositätserhöhung von mindestens 100 % gegenüber einer Lösung dieses Aminosäuretensids oder der Tenside in Wasser zeigt.

Die in den Unteransprüchen beschriebenen Merkmale betreffen bevorzugte Weiterbildungen des erfindungsgemäßen Reinigungs- und Pflegemittels bzw. des erfindungsgemäßen Verfahrens.

Nachfolgend wird die vorliegende Erfindung anhand von Ausführungsbeispielen unter Bezugnahme auf die anliegenden graphischen Darstellungen näher erläutert. Dabei zeigen:
Figur 1 ein Diagramm, in dem die Auslaufzeit ALZ einer Mischung von Natrium Cocoyl Sarkosinat 3 % WAS und Cocamidopropylbetain 3,8 % WAS in Sekunden abhängig vom pH-Wert dargestellt ist;
Figur 2 ein Diagramm, in dem die Auslaufzeit ALZ von Natrium Cocoyl Sarkosinat allein, 10 % WAS, in Sekunden abhängig vom pH-Wert dargestellt ist;
Figur 3 ein Vergleichsdiagramm, in dem der pH-abhängige Viskositätsverlauf von Wasser dargestellt ist;
Figur 4 ein Vergleichsdiagramm, in dem der pH-abhängige Viskositätsverlauf von Cocamidopropylbetain, 10 % WAS, als Auslaufzeit in Sekunden dargestellt ist;
Figur 5 ein Diagramm, in dem die Auslaufzeit ALZ von Dinatrium Cocoyl Glutamat allein, 10 % WAS, in Sekunden abhängig vom pH-Wert dargestellt ist;
Figur 6 ein Diagramm, in dem die Auslaufzeit ALZ von Natrium Cocoyl Alaninat allein, 10 % WAS, in Sekunden abhängig vom pH-Wert dargestellt ist;
Figur 7 ein Diagramm, in dem die Auslaufzeit ALZ einer Mischung von Dinatrium Cocoyl Glutamat 3 % WAS und Lauraminoxid 3 % WAS in Sekunden abhängig vom pH-Wert dargestellt ist;
Figur 8 ein Diagramm, in dem die Auslaufzeit ALZ einer Mischung von Natrium Cocoyl Alaninat 3 % WAS und Lauraminoxid 3 % WAS in Sekunden abhängig vom pH-Wert dargestellt ist;
Figur 9 ein Diagramm, in dem die Auslaufzeit ALZ einer Mischung von Natrium Cocoyl Glycinat 3 % WAS und Lauraminoxid 3 % WAS in Sekunden abhängig vom pH-Wert dargestellt ist;
Figur 10 ein Vergleichsdiagramm, in dem der pH-abhängige Viskositätsverlauf von Lauraminoxid, 10 % WAS, als Auslaufzeit in Sekunden dargestellt ist;
Figur 11 ein Vergleichsdiagramm, in dem der pH-abhängige Viskositätsverlauf von Natrium Laurethsulfat, 10 % WAS, als Auslaufzeit in Sekunden dargestellt ist;
Figur 12 ein Vergleichsdiagramm, in dem der pH-abhängige Viskositätsverlauf von Coco-Glucosid, 10 % WAS, als Auslaufzeit in Sekunden dargestellt ist;
Figur 13 ein Diagramm, in dem die Auslaufzeit ALZ einer Mischung von Natrium Cocoylsarkosinat 3 % WAS und Lauraminoxid 3 % WAS in Sekunden abhängig vom pH-Wert dargestellt ist;
Figur 14 ein Diagramm, in dem die Auslaufzeit ALZ einer Mischung von Natrium Cocoylsarkosinat 3 % WAS und Natrium Laurethsulfat 2,8 % WAS in Sekunden abhängig vom pH-Wert dargestellt ist;
Figur 15 ein Diagramm, in dem die Auslaufzeit ALZ einer Mischung von Natrium Cocoylsarkosinat 3 % WAS und Coco-Glucosid 3,5 % WAS in Sekunden abhängig vom pH-Wert dargestellt ist;
Figur 16 ein Diagramm, in dem die Auslaufzeit ALZ einer Mischung von Natrium Cocoylsarkosinat 3 % WAS, Cocamidopropylbetain 1,9 % WAS und Lauraminoxid 1,5 % WAS in Sekunden abhängig vom pH-Wert dargestellt ist;
Figur 17 ein Diagramm, in dem die Auslaufzeit ALZ einer Mischung von Natrium Cocoylsarkosinat 3 % WAS, Cocamidopropylbetain 1,9 % WAS und Natrium Laurethsulfat 1,4 % WAS in Sekunden abhängig vom pH-Wert dargestellt ist;
Figur 18 ein Diagramm, in dem die Auslaufzeit ALZ einer Mischung von Natrium Cocoylsarkosinat 3 % WAS, Lauraminoxid 1,5 % WAS und Natrium Laurethsulfat 1,4 % WAS in Sekunden abhängig vom pH-Wert dargestellt ist;
Figur 19 ein Diagramm, in dem die Auslaufzeit ALZ einer Mischung von Natrium Cocoylsarkosinat 3 % WAS, Cocamidopropylbetain 1,9 % WAS und Coco-Glucosid 1,75 % WAS in Sekunden abhängig vom pH-Wert dargestellt ist;
Figur 20 ein Diagramm, in dem die Auslaufzeit ALZ für Natriumlaurethsulfat (10 % WAS) und C8-C22-Alkylglukosid (5 % WAS) und Natriumlauroylglutamat (2 % WAS) und Cocamidopropylbetain (3,2 % WAS) abhängig vom pH-Wert dargestellt ist;
Figur 21 ein Diagramm, in dem die Auslaufzeit ALZ für Natriumlaurethsulfat (10 % WAS) und C8-C22-Alkylglukosid (5 % WAS) und Cocamidopropylbetain (3,2 % WAS) abhängig vom pH-Wert dargestellt ist;
Figur 22 ein Diagramm in dem die Auslaufzeit ALZ für Cocamidopropylbetain (5,15 % WAS), Natriumlaurethsulfat (5,8 % WAS) und Natriumlauroylsarkosinat (0,5 % WAS) abhängig vom pH-Wert dargestellt ist;
Figur 23 zeigt ein Diagramm, in dem die **Auslaufzeit** ALZ von Dinatriumcocoylglutamat allein, 9 % WAS, in Sekunden abhängig vom pH-Wert dargestellt ist;
Figur 24 zeigt ein Diagramm, in dem die Auslaufzeit ALZ von Natriumlauroylsarkosinat allein, 9 % WAS, in Sekunden abhängig vom pH-Wert dargestellt ist. Wie auch bei Natriumcocoylsarkosinat tritt ein weiteres Viskositätsmaximum bei pH 3,5 auf, an dem das Produkt weiß gelig ist;
Figur 25 zeigt ein Diagramm, in dem die Auslaufzeit ALZ von Natriumcocoylalaninat allein, 9 % WAS, in Sekunden abhängig vom pH-Wert dargestellt ist;
Figur 26 zeigt ein Diagramm, in dem die Auslaufzeit ALZ von Natriumacylglutamat allein, 9 % WAS, in Sekunden abhängig vom pH-Wert dargestellt ist;
Figur 27 zeigt ein Diagramm in dem die Auslaufzeit ALZ für Cocamidopropylbetain (5,15 % WAS) und Natriumlaurethsulfat (5,8 % WAS) abhängig vom pH-Wert dargestellt ist.

### Vergleich der Viskositäten in ALZ [s] und mPas bzw. cP:

**Vergleichswerte Viskositäten**

| mPas | ALZ [s] | |
|---|---|---|
| 1 | 5 | Beispiel Wasser |
| 1000 | 30 | |
| 2000 | 60 | |
| 2500 | 70 | |
| 3360 | 88 | (Eichmessung) |
| 3700 | 100 | |
| 4200 | 114 | (Eichmessung) |
| 4300 | 130 | |
| 4900 | 180 | |
| 5100 | 200 | |
| 5200 | 214 | (Eichmessung) |
| 5300 | 220 | |

| | | |
|---|---|---|
| 1 mPas entspricht 1 cP | | |

Für die Eichmessungen wurden tensidische Produkte unterschiedlicher Viskosität in einem Rotationsviskosimeter gemessen und anschließend die Auslaufzeit im Auslaufbecher bestimmt. Die anderen Werte sind an diese Messungen angenähert. Handelsübliche Produkte beginnen bei 3000 mPas/ ca. 80 s ALZ bis 10000 mPas / > 400 s ALZ.

Unerwarteter Weise wurde bei Versuchen zur Herstellung milder Duschbadformulierungen mit waschaktiven Substanzen wie beispielsweise Natrium Lauroylsarkosinat, Natrium Cocoylglutamat und Cocamidopropylbetain gefunden, dass bei der Einstellung des pH-Wertes in den pH-hautneutralen Bereich Viskositätserhöhungen auftraten, die bei weiterer Säurezugabe wieder verschwanden.

Es wurden Versuche durchgeführt, in denen die Viskositäten von Lösungen verschiedener Tenside und Tensidgemische in Abhängigkeit vom pH-Wert gemessen wurden. Dazu wurde jeweils mit einem DIN-Auslaufbecher 4 mm / 50 Gramm Produkt bei 20 °C gemessen. In oben stehender Tabelle ist ein Vergleich zwischen Viskositäten in mPas und s ALZ zu finden.

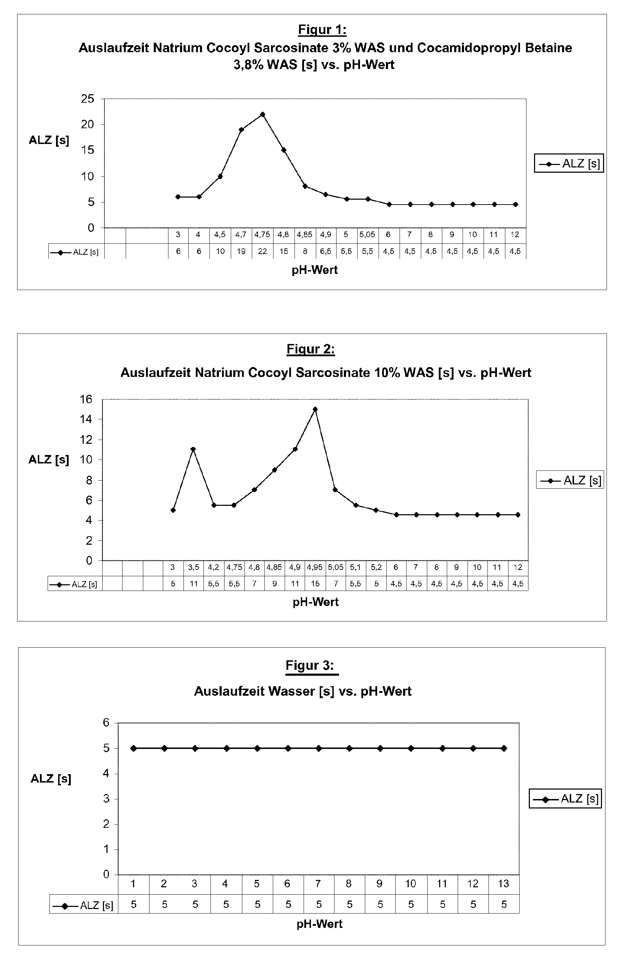

Die Tensidlösungen wurden mit entmineralisiertem Wasser nach VDE 0510, DIN 43530 angesetzt und die pH-Einstellungen mit KOH 10 %ig und Ameisensäure 85 %ig bzw. 8,5 %ig durchgeführt. Die Darstellung der Ergebnisse, die sich in den Diagrammen gemäß den Figuren 1 bis 27 findet, ist im x-Achsenbereich nicht immer maßstabsgetreu. Der betrachtete Messbereich war in der Regel pH 3 bis pH 12, über diesen Bereich hinaus nur in Ausnahmefällen. In den extremen pH-Bereichen ist aber auch kein erfindungsgemäßer Effekt zu erwarten.

Bei der Ausarbeitung des Effektes wurde gefunden, dass beispielsweise 10 %ige Lösungen von Natrium Lauroylsarkosinat, Natrium Cocoylsarkosinat bzw. Natrium Myristoylsarkosinat einen deutlichen Viskositätsanstieg bei ca. pH 5,0 zeigen (Bei geringer konzentrierten Lösungen ist der Effekt entsprechend schwächer ausgeprägt.) Diese pH-Wert abhängige Verdickung gering konzentrierter Lösungen des Einzelrohstoffes wurde dann auch bei Natrium Cocoylglutamat und anderen Aminosäuretensiden gefunden. Natrium Cocoylglutamat ist ein Acylderivat einer Dicarbonsäure und kann daher pH-abhängig als Natrium-oder Dinatriumverbindung vorliegen; Natrium Cocoylglutamat bezeichnet im nachfolgenden Text beide möglichen Formen.

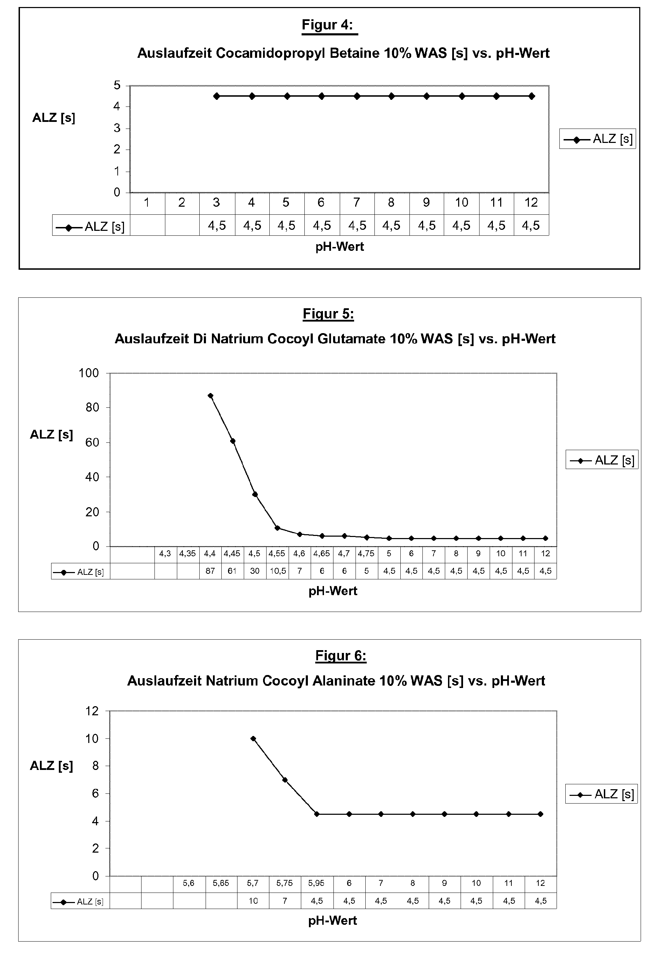

Figur 1 zeigt ein Diagramm, in dem die Auslaufzeit ALZ einer Mischung von Natrium Cocoylsarkosinat 3 % WAS und Cocamidopropylbetain 3,8 % WAS dargestellt ist. Figur 2 ein Diagramm, in dem die Auslaufzeit ALZ von Natrium Cocoylsarkosinat allein, 10 % WAS, in Sekunden abhängig vom pH-Wert dargestellt ist. Zum Vergleich wurden in den Figuren 3 und 4 die pH-Wert-abhängigen Viskositätsverläufe von Wasser und Cocamidopropylbetain allein dargestellt. Die Diagramme zeigen, dass es hier keinerlei Viskositätsänderung über den gesamten betrachteten pH-Bereich gibt.

Diese bislang unbekannte pH-Wert-abhängige Verdickung vergleichsweise gering konzentrierter Lösungen des genannten Tensids wurde auch bei Natrium Cocoylglutamat und anderen Aminosäuretensiden gefunden. Im Unterschied zu den Acylsarkosinat-Lösungen zeigen wie sich aus den Figuren 5 und 6 ergibt die Cocoylglutamat- und Cocoylalaninat-Verdünnungen Viskositätserhöhungen, die über viskose, klare Lösungen in weiße, pastös-feste Produkte übergehen (in den Graphiken sind feste Mischungen mit pH-Angaben ohne Viskositätsangabe gekennzeichnet.) Deshalb gibt es in den Figuren 5 und 6 kein Viskositätsmaximum. Viskose Cocoylglycinat-Lösungen sind nicht darstellbar, das Glycinat flockt bei pH-Erniedrigung zunehmend aus der Lösung aus.

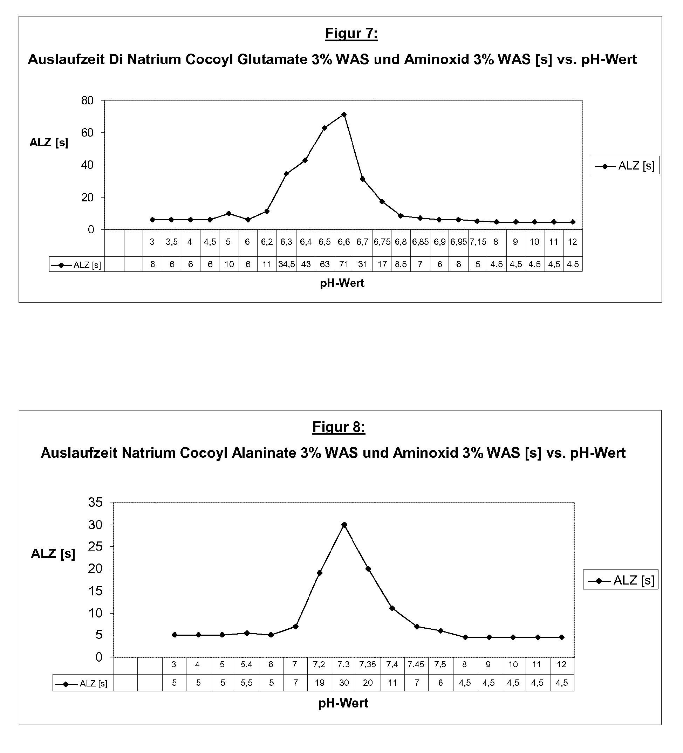

Dagegen ist es überraschend möglich, synergistische Mischungen von Dinatriumcocoylglutamat, Natriumcocoylalaninat und sogar Natriumcocoylglycinat mit dem nichtionischen bzw. zwitterionischen Tensid Lauraminoxid herzustellen, wie die Figuren 7, 8 und 9 verdeutlichen.

Dies ist umso erstaunlicher, da ein derartiger pH-Verdickungseffekt in der Literatur bei diesen Tensiden nicht beschrieben wird, obwohl diese Tenside an sich bekannt sind und häufig verwendet werden.

In der nachfolgenden Tabelle sind gemessene pH-Wert-abhängige Auslaufzeiten (ALZ) von Aminosäuretensiden sowie Mischungen dieser Verbindungen mit Aminoxid bei verschiedenen Mengen an waschaktiver Substanz zusammengefasst, um die pH-Wert-abhängige Viskositätserhöhung zu verdeutlichen:

| ALZ [s] | pH 3 | pH 4 | pH 5 | pH 6 | pH 7 | pH 8 | Viskositätserhöhung (10% WAS) | Viskositätserhöhung (6% WAS) |
|---|---|---|---|---|---|---|---|---|
| Natrium Cocoyl Sarkosinat 10% WAS | 5 | 6 | 15 (4,95) | 4,5 | 4,5 | 4,5 | 300% | |
| Natrium Cocoyl Sarcosinate 3% WAS und Lauramine Oxide 3% WAS | 6 | 6 | 6 | 6 | 36 (7,05) | 4,5 | | 600% |
| Di Natrium Cocoyl Glutamate 10% WAS | n. b. | 30 (4,45) | 4,5 | 4,5 | 4,5 | 4,5 | ca. 600% | |
| Di Natrium Cocoyl Glutamate 3% WAS und Lauramine Oxide 3% WAS | 6 | 6 | 10 | 71 (6,6) | 6 (6,95) | 4,5 | | > 1000% |
| Natrium Cocoyl Alaninate 10% WAS | n. b. | n. b. | 10 (5,7) | 4,5 | 4,5 | 4,5 | ca. 200% | |
| Natrium Cocoyl Alaninate 3% WAS und Lauramine Oxide 3% WAS | 5 | 5 | 5 | 5 | 30 (7,3) | 4,5 | | 600% |
| Natrium Cocoyl Glycinate 10% WAS | n. b. | n. b. | n. b. | n. b. | n. b. | n. b. | n. b. | |
| Natrium Cocoyl Glycinate 3% WAS und Lauramine Oxide 3% WAS | n. b. | n. b. | 11 | 4,5 | 11 (7,4) | 4,5 | | ca. 200 % |

| ALZ [s] | pH 3 | pH 4 | pH 5 | pH 6 | pH 7 | pH 8 | | Viskositätserhöhung (10% WAS) |
|---|---|---|---|---|---|---|---|---|
| Natrium Cocoyl Sarkosinat 10% WAS | 5 | 6 | 15 (4,95) | 4,5 | 4,5 | 4,5 | | 300% |
| Di Natrium Cocoyl Glutamate 10% WAS | n. b. | 30 (4,45) | 4,5 | 4,5 | 4,5 | 4,5 | | ca. 600% |
| Natrium Cocoyl Alaninate 10% WAS | n. b. | n. b. | 10 (5,7) | 4,5 | 4,5 | 4,5 | | ca. 200% |
| Natrium Cocoyl Glycinate 10% WAS | n. b. | n. b. | n. b. | n. b. | n. b. | n. b. | | n. b. |

| ALZ [s] | pH 3 | pH 4 | pH 5 | pH 6 | pH 7 | pH 8 | | Viskositätserhöhung (6% WAS) |
|---|---|---|---|---|---|---|---|---|
| Natrium Cocoyl Sarcosinate 3% WAS und Lauramine Oxide 3% WAS | 6 | 6 | 6 | 6 | 36 (7,05) | 4,5 | | 600% |
| Di Natrium Cocoyl Glutamate 3% WAS und Lauramine Oxide 3% WAS | 6 | 6 | 10 | 71 (6,6) | 6 (6,95) | 4,5 | | > 1000% |
| Natrium Cocoyl Alaninate 3% WAS und Lauramine Oxide 3% WAS | 5 | 5 | 5 | 5 | 30 (7,3) | 4,5 | | 600% |
| Natrium Cocoyl Glycinate 3% WAS und Lauramine Oxide 3% WAS | n. b. | n. b. | 11 | 4,5 | 11 (7,4) | 4,5 | | ca. 200 % |

Durch umfangreiche Versuche im Rahmen der vorliegenden Erfindung konnte gezeigt werden, dass Partialneutralisations- bzw. Puffergemische aus elektrisch neutralen bzw. isoelektrischen bzw. neutralisierten anionischen Tensiden und freien anionischen Tensiden der gleichen Verbindung bzw. des gleichen Rohstoffes in einem Rohstoff-spezifischen (speziellen), engen pH-Bereich einen ungewöhnlich starken Viskositätsaufbau bei ungewöhnlich geringen WAS-Konzentrationen zeigen, ohne Verwendung von zusätzlichen externen Verdickern.

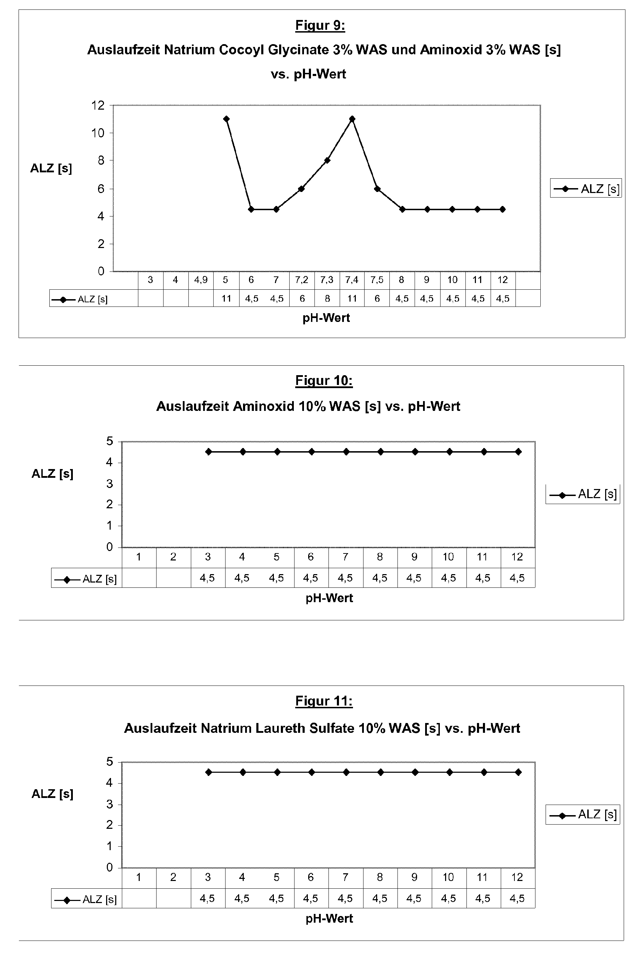

Es folgen weitere Beispiele zur Erläuterung mit Natrium Cocoylsarkosinat in Mischung mit einem nichtionischen/zwitterionischen Tensid einem nicht pH-sensitiven anionischen Tensid und einem nichtionischen Tensid:
Lauraminoxid, das schon in synergistischer Mischung mit verschiedenen Aminosäuretensiden betrachtet wurde (s.o.) zeigt als Einzelsubstanz keinerlei Viskositätsänderung über den gesamten betrachteten pH-Bereich, wie das Diagramm gemäß Figur 10 belegt. Lauraminoxid ist ein nichtionisches/zwitterionisches Tensid, das bei sauren pH-Werten auch kationische Eigenschaften haben kann.

Gleiches gilt für nicht pH-sensitive anionische Tenside wie Natrium Laurethsulfat, wie das Diagramm gemäß Figur 11 verdeutlicht.

Gleiches gilt für nichtionische Tenside wie Coco-Glucosid, wie das Diagramm gemäß Figur 12 verdeutlicht.

Mischungen von Natrium Cocoylsarkosinat mit dem nichtionischen/zwitterionischen Tensid Lauraminoxid bzw. dem anionischen Tensid Natrium Laurethsulfat bzw. dem nichtionischen Tensid Coco-Glucosid zeigen jedoch synergistische Viskositätseffekte, überraschender Weise aber bei unterschiedlichen pH-Werten und auch verschieden zu der synergistischen Mischung mit Cocamidopropylbetain. Dies belegen die Diagramme gemäß den Figuren 13, 14 und 15.

Die gemessenen Viskositätsmaxima von Natrium Cocoylsarkosinat bei pH 7,05 (in Mischung mit nichtionischem/zwitterionischem Tensid) und pH 4,2 (in Mischung mit nicht pH-sensitivem anionischen Tensid) sind in überraschender Weise deutlich verschieden zum Viskositätsmaximum von pH 4,95 in Mischung mit amphoterem Tensid und können derzeit mechanistisch im Gegensatz zum pH-Wert von 4,95 (Auftreten von unlöslicher Sarkosinsäure bzw. Puffergemische aus elektrisch neutralen bzw. isoelektrischen bzw. neutralisierten anionischen Tensiden und freien anionischen Tensiden der gleichen Verbindung) nicht erklärt werden. Natrium Laurethsulfat dient hier nur der Darstellung des Effektes von Sarkosinat in Mischung mit einem nicht pH-sensitiven anionischen Tensid, in den neuen Formulierungskonzepten ist dieses ethoxylierte Tensid eher unerwünscht, es wird aber dennoch, wie ausgeführt, weit verbreitet eingesetzt. Auch das gemessene Viskositätsmaximum von Natrium Cocoylsarkosinat bei pH 4,45 in Mischung mit nichtionischen Tensid Coco-Glucosid ist deutlich unterhalb des Viskositätsmaximums von 4,95 von Natrium Cocoylsarkosinat und der Effekt nicht abschließend erklärbar.

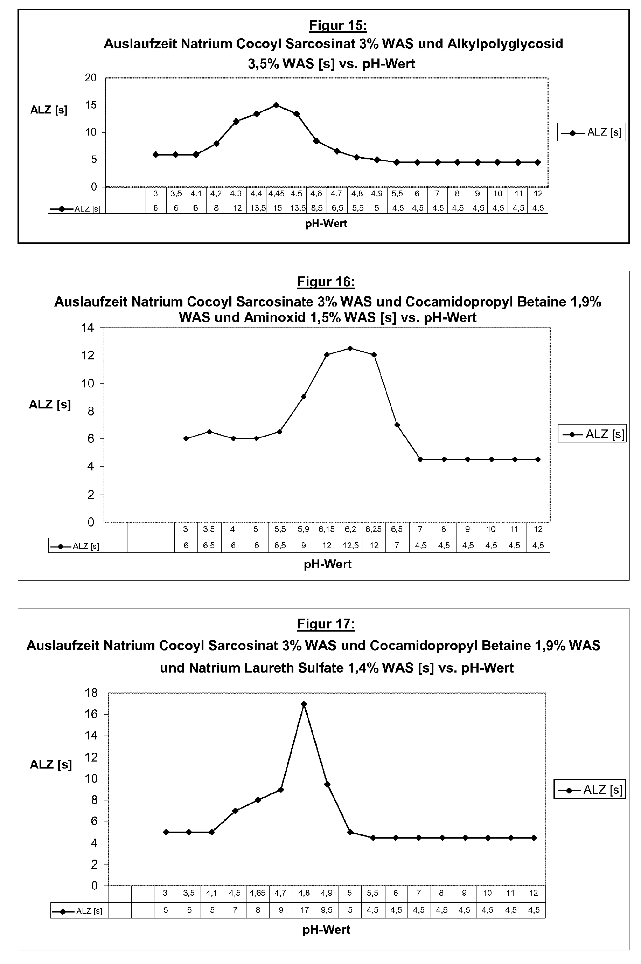

Die im Rahmen der vorliegenden Erfindung gefundenen Erkenntnisse erlauben es dem Formulierer durch geschickte Wahl der Tensid-Mischung den pH-Wert in den gewünschten Bereich zu verschieben, wie beispielsweise das Diagramm von Figur 16 zeigt. Eine Mischung von Cocoylsarkosinat mit Cocamidopropylbetain und Lauraminoxid zeigt ein Viskositätsmaximum bei pH 6,2. Zu erwarten wäre dieses Viskositätsmaximum aus den binären Mischungen jedoch bei ca. pH 5,9.

Wie sich Figur 17 entnehmen lässt, zeigt eine Mischung von Natrium Cocoylsarkosinat mit Cocamidopropylbetain und Natrium Laurethsulfat ein Viskositätsmaximum bei pH 4,8. Zu erwarten wäre dieses Viskositätsmaximum aus den binären Mischungen jedoch bei ca. pH 4,6.

Liegen die Viskositätsmaxima jedoch zu weit auseinander, resultiert nur noch ein geringer synergistischer Effekt: Wie sich aus Figur 18 ergibt zeigt eine Mischung aus Natrium Cocoylsarkosinat mit Lauraminoxid und Natrium Laurethsulfat ein geringes Viskositätsmaximum bei pH 6,05. Zu erwarten wäre dieses Viskositätsmaximum aus den binären Mischungen bei ca. pH 5,5. Diese Mischung wie andere Sarkosinat-Lösungen zeigt eine Auffälligkeit bei ca. pH 3,5, auf die aber nicht näher eingegangen werden soll, da sie derzeit keine kommerzielle Verwendung bietet.

Mischungen von Natrium Cocoylsarkosinat mit dem nichtionischen Tensid Alkylpolyglykosid und dem amphoteren Tensid Cocamidopropylbetain zeigen ebenfalls synergistische Viskositätseffekte, in diesem Fall wie erwartet bei pH 4,7. Dies belegt das Diagramm gemäß Figur 19.

Der Viskositätsaufbau ist bei diesen Basisformulierungen damit je nach Aminosäuretensid unterschiedlich stabil, führt je nach Rohstoff ab bestimmten WAS-Konzentrationen und pH-Werten zu einem klaren über trüb-durchscheinenden bis weiß-trüben viskosen Produkt. In vielen Fällen sind synergistische Mischungen mit anderen Tensiden möglich, aus denen neue, bislang unbekannte Formulierungskonzepte entwickelt wurden.

Die vorliegende Erfindung ist damit grundsätzlich anwendbar für alle Reinigungs- und Pflegemittel, vorzugsweise Haar- und Körperreinigungsprodukte und Haar- und Körperpflegeprodukte, Oberflächen- und sonstige Reinigungs- und/oder Pflegemittel als viskose, wässrige oder andere Lösung aus Mischungen von freien und Neutralisationsprodukten spezieller anionischer Tenside (Einem anionischen Tensid oder mehreren) bei speziellen pH-Werten ohne jeden weiteren Zusatzstoff, also eine Mischung aus anionischem Tensid/anionischen Tensiden, einem pH-Regulator und Wasser. Eine Änderung des spezifischen pH-Wertes des engen Bereiches des Viskositätsmaximums der Mischung führt zu einer deutlichen Viskositätsverringerung.

Die Erfindung ist insbesondere anwendbar bei Reinigungs- und Pflegemitteln aus Mischungen mit wenigstens einem erfindungsgemäßen pH-sensitiven Tensid und mit einem oder mehreren amphoteren Tensid bzw. Tensiden, die synergistisch sein können.

Dies können insbesondere auch Mischungen des pH-sensitiven Tensids mit einem oder mehreren anionischen Tensid bzw. Tensiden ohne den erfindungsgemäßen pH-Effekt sein, die ebenfalls synergistisch sein können.

Ebenfalls erfindungsgemäß sind Mischungen wenigstens eines pH-sensitiven Tensids mit einem oder mehreren nichtionischen Tensid bzw. Tensiden, die ebenfalls synergistisch sein können.

Ebenfalls erfindungsgemäß sind Mischungen wenigstens eines pH-sensitiven Tensids mit einem oder mehreren kationischen Tensid bzw. Tensiden, die ebenfalls synergistisch sein können.

Vorzugsweise wird der pH-Wert mit einem oder mehreren pH-Regulatoren, jedoch bevorzugt je nach Ausgangs-pH-Wert mit organischen Säuren oder anorganischen Basen wie Kaliumhydroxid eingestellt.

Die Erfindung betrifft insbesondere Reinigungs- und Pflegemittel, bei denen das Mittel auf einen rohstoff- und formulierungsspezifischen pH-Wert von 2,0 bis 10,0; vorzugsweise 4,0 bis 7,0 und für die Kosmetik besonders bevorzugt auf 4,1 bis 5,8 eingestellt wird.

Die Erfindung betrifft insbesondere Reinigungs- und Pflegemittel, bei denen das anionische Tensid oder die anionischen Tenside bevorzugt aus der Gruppe der sogenannten Aminosäuretenside oder analogen Verbindungen stammen.

Die Erfindung betrifft insbesondere Reinigungs- und Pflegemittel, bei denen das anionische Tensid oder die anionischen Tenside aus der Gruppe der sogenannten Aminosäuretenside, bevorzugt aus Vertretern der Sarkosinate, Glutamate, Glycinate oder Alaninate bzw. deren Mischungen bestehen.

Die Erfindung ist auch anwendbar bei allen anionischen Tensiden, die ebenfalls einen erfindungsgemäßen Viskositätseffekt zeigen.

Die Erfindung betrifft insbesondere Reinigungs- und Pflegemittel, bei denen das anionische Tensid oder die anionischen Tenside bevorzugt aus der Gruppe der sogenannten Aminosäuretenside sind und diese bevorzugt in Mischung mit einem oder mehreren Tensiden aus der Gruppe der amphoteren und/oder anionischen (ohne den erfindungsgemäßen pH-Effekt) und/oder nichtionischen und/oder kationischen Rohstoffen formuliert werden.

Die Erfindung betrifft insbesondere Reinigungs- und Pflegemittel in Form von Duschbädern/Flüssigseifen/Shampoos/Schaumbädern oder andere Reinigungs- und Pflegemittel, welche vorzugsweise die nachfolgenden Mengenverhältnisse (Gew.% bzw. Aktivgehalte) aufweisen:

**Anionisches Tensid mit erfindungsgemässem pH-Effekt wie**

| | | | | | | | |
|---|---|---|---|---|---|---|---|
| Natrium Lauroylsarcosinat | 0,01% | - | 50%, | bevorzugt | 2% | - | 5%; |
| amphoteres Tensid wie | | | | | | | |
| Cocamidopropylbetain | 0% | - | 50%, | bevorzugt | 3% | - | 8%; |
| | | | | | | | |
| anionisches Tensid ohne erfindungsgemässen pH-Effekt wie Natrium Laureth Sulfat | 0% | - | 30%, | bevorzugt | 0% | - | 5%; |
| | | | | | | | |
| nichtionisches Tensid wie Cocoglucoside | 0% | - | 30%, | bevorzugt | 0% | - | 5%; |
| | | | | | | | |
| kationisches Tensid wie Behentrimoniumchlorid | 0% | - | 30%, | bevorzugt | 0% | - | 3%; |
| | | | | | | | |
| Polymere (geladen und ungeladen) | 0% | - | 20%, | bevorzugt | 0% | - | 3%; |
| | | | | | | | |
| Parfüm | 0% | - | 10%, | bevorzugt | 0% | - | 1%; |
| | | | | | | | |
| Zusätzliche(s) KonservierungsMittel (Formulierungsabhängig) | 0% | - | 10%, | bevorzugt | 0,3% | - | 1%; |
| Alle sonstigen Wirk- und/oder | | | | | | | |
| Hilfsstoffe (Pflanzenextrakte, | | | | | | | |
| Perlglanz, Farbstoffe, | | | | | | | |
| Silicone, Elektrolyte, Reibmittel, Füllstoffe usw.) | | | | | | 0% - | 99% |
| | | | | | | | |
| pH-Regulator (einer oder | | | | | | | |
| mehrere) | | 0,001% - | | 30%, | | rohstoff- und rezepturspezifisch; | |
| Wasser ad 100, | | | | | | | |

wobei das Mengenverhältnis pH-sensitives anionisches Tensid zu Cotensid beispielsweise 1 : 10 bis 10 : 1 betragen kann. In der Tendenz ist wirtschaftlich eher eine geringere Konzentration an Sarkosinat oder Glutamat etc. im Vergleich zu den anderen formulierten Tensiden bevorzugt.

Die vorliegende Erfindung beschreibt somit Reinigungs- und Pflegemittel, welche ökologisch, dermatologisch und sensorisch deutlich vorteilhaft sind gegenüber aktuellen Formulierungen nach dem Stand der Technik.

Bei dem erfindungsgemäßen Reinigungs- und Pflegemittel kann das Produkt durch Zugabe eines oder weiterer Inhaltsstoffe aus der Gruppe der Wirk- und/oder Hilfsstoffe wie z. B. Elektrolyte und/oder Polymere stabilisiert werden.

Das erfindungsgemäße Reinigungs- und Pflegemittel wird eingesetzt in Verfahren zur Reinigung und/oder Pflege von Oberflächen und/oder Geweben/Textilien, wobei das Mittel auf eine Oberfläche aufgebracht wird und nach einer gegebenen Einwirkzeit bevorzugt, aber nicht zwangsläufig, mit Wasser bzw. wässrigen Lösungen wieder abgespült wird.

### Beispiele für erfindungsgemäße pH-sensitive anionische Aminosäuretenside:

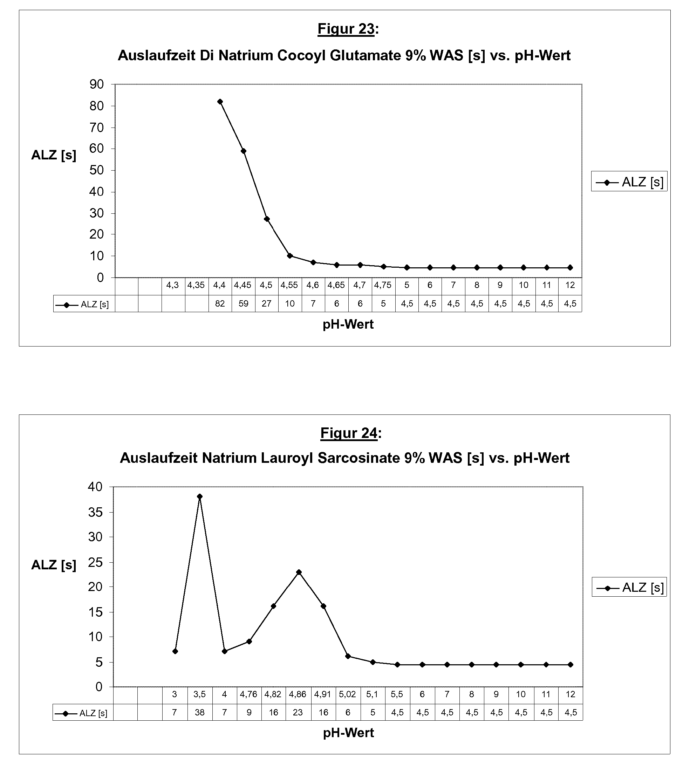

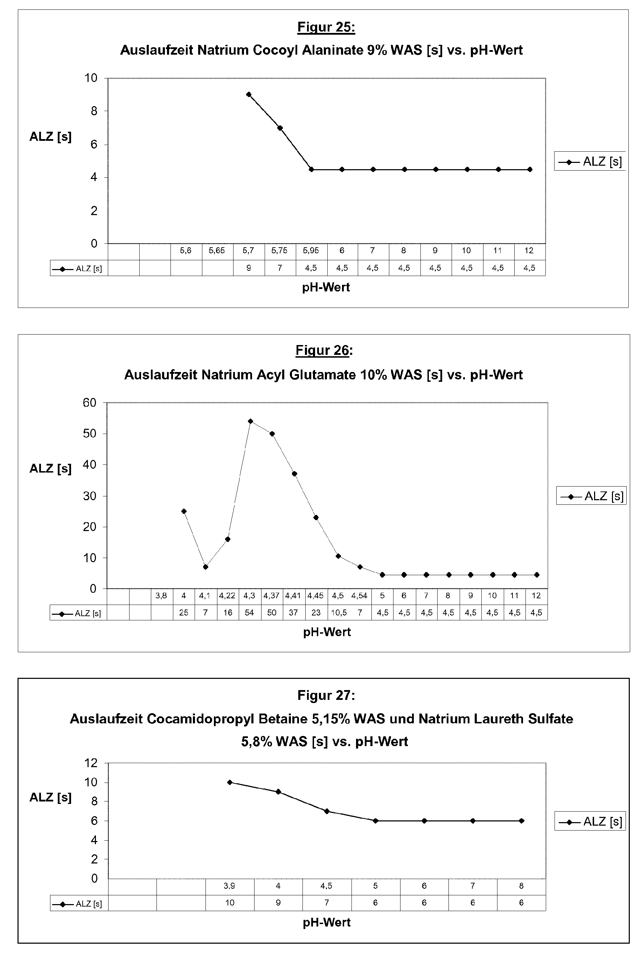

I:
   30 % Dinatrium Cocoylglutamat (30%ig, Hersteller Ajinomoto)
   70% Wasser
   Organische Säuren zur Einstellung pH etwa 4,25
   (Aussehen klar, später Eintrübung)
   Viskositätsverlauf siehe Figur 23.
   Figur 23 zeigt ein Diagramm, in dem die Auslaufzeit ALZ von Dinatrium Cocoyl Glutamat allein, 9 % WAS, in Sekunden abhängig vom pH-Wert dargestellt ist;
II:
   30 % Natrium Lauroylsarkosinat (30%ig, Hersteller Croda)
   70% Wasser
   Organische Säuren zur Einstellung pH etwa 4,95
   (Aussehen trüb); Viskositätsverlauf siehe Figur 24;
III:
   30 % Natrium Cocoylalaninat (30 %ig, Hersteller Ajinomoto)
   70% Wasser
   Organische Säuren zur Einstellung pH etwa 5,5
   (Aussehen weiß und fest); Viskositätsverlauf siehe Figur 25;
IV:
   20 % Natrium Acylglutamat (50%ig, Hersteller Cognis)
   80% Wasser
   Organische Säuren zur Einstellung pH etwa 4,5
   (Aussehen weiß, später fest); Viskositätsverlauf siehe Figur 26.

Der Effekt der pH-abhängigen Viskositätsveränderung ist umso überraschender, als die anionischen Tenside, bei denen dieses Verhalten bislang gefunden wurde, alle der Gruppe der Aminosäure-basierten Tenside oder analogen Verbindungen (z. B. Sarkosinate) entstammen, die allgemein als Basistensid schwer bis nicht verdickbar (ohne zusätzlichen Verdicker) sind.

Andere anionische Tenside wie oben gezeigt Natrium Laurethsulfat, aber auch Alkansulfonate, Olefinsulfonate, Vertreter der Taurate (einer Sulfonsäuremodifizierten Aminosäureverbindung), Isethionate, Amphoacetate oder Sulfosuccinate zeigten bei entsprechenden Versuchen keine Viskositätserhöhung im pH-Bereich von pH 12 alkalisch über pH 7 neutral bis ca. pH 2 bis 3 im sauren Bereich.

Noch überraschender ist, dass der Viskositätsaufbau z. B. bei Natrium Lauroylsarkosinat in einem pH-Bereich erfolgt, der im weitesten Sinne als pH-neutral bis pH-Hautneutral bezeichnet werden kann und damit für Anwendungen im Bereich der menschlichen Haut sehr geeignet ist. Damit ist auch eine milde und marktübliche Konservierung mit organischen Säuren wie beispielsweise Ameisensäure möglich. Die Zugabe von einem oder mehreren Cotensiden erlaubt je nach Rohstoff und Menge wie gezeigt sogar eine Verschiebung des pH-Wertes des Viskositätsmaximums.

Bei den erfindungsgemäßen Formulierungen unter Verwendung milder AminosäureTenside mit z. B. Cocamidopropylbetain oder anderen Cotensiden wurden überraschenderweise synergistische Effekte gefunden: Diese Mischungen waren ebenfalls vor der pH Wert-Einstellung dünnviskos, zeigten dann bei einem ganz speziellen pH-Wert ein deutlich wahrnehmbares Viskositätsmaximum und wurden dann bei weiterer pH-Wert Veränderung wieder dünnflüssig. Dies alles unerwarteterweise bei für diese Tensidklassen sehr geringen WAS-Konzentrationen (Zum Beispiel Cocamidoproylbetain ca. 4% und Natrium Lauroylsarkosinat ca. 3%). Synergistische Mischungen mit weiteren, breit in der Kosmetik eingesetzten Cotensiden führen damit überraschenderweise zu stabilen, marktfähigen Produkten und neuen, bislang unbekannten Produktkonzepten, auch im Falle des Natrium Cocoylglutamat, das, ohne Co-Tenside formuliert, nicht lagerstabil ist.
So konnten exemplarisch Gemische von Sarkosinaten und/oder Glutamaten mit anderen amphoteren, anionischen, nichtionischen und überraschenderweise kationischen Rohstoffen dargestellt werden, die in einem spezifischen, engen (wie oben beschriebenen) pH-Wertbereich eine hohe, markt- und produktübliche (z. Beispiel Duschbäder) Viskosität aufbauen können.

Noch überraschender gelang es am System Cocamidopropylbetain - Natrium Lauroylsarkosinat zu zeigen, dass dieser Effekt a) reproduzierbar ist, b) das Viskositätsmaximum und damit der pH Wert Langzeit lagerstabil ist (> 1 a), c) der Effekt reversibel ist in Abhängigkeit der entstehenden Elektrolytkonzentration, d. h. der pH-Wert mehrfach angehoben bzw. abgesenkt werden konnte und d) eine marktgängige Viskosität für Duschbäder o. ä. zu erreichen ist.

So zeigen beispielsweise Rezepturen für den Preiseinstiegsbereich aus Cocamidopropylbetain und Natrium Lauroylsarkosinat mit ca. 6 % Gesamtaktivgehalt in einem speziellen pH-Bereich (Mischungsabhängig zwischen pH 4,5 und 4,95) einen guten Viskositätsaufbau, der den Viskositätsaufbau einer exemplarischen Mischung von 5% Natrium Laureth Sulfat mit 1 % Cocamidopropylbetain mit 3 - 4 % Natrium Chlorid (alles Aktivgehaltsangaben) übertraf bei einer besseren Sensorik. Die Natrium Laurethsulfat/ Cocamidopropylbetain Mischung zeigte eine schleimige fädenziehende Sensorik mit verminderter Schaumentwicklung durch eine sich nur langsam lösende Gelstruktur; Nachteile ergeben sich für diese Mischung auch in der Augenschleimhautverträglichkeit durch die hohe Salzkonzentration.
Die erfindungsgemäße Mischung aus Cocamidopropylbetain und Natrium Lauroylsarkosinat hat hingegen eine angenehme, noch marktübliche Sensorik bei mittlerer Viskosität und ein deutlich stärkeres Schaumvermögen, das zudem, wie bei den milden Tensiden üblich, eher feinporig ist und ein angenehmes Hautgefühl hinterläßt. Zugabe von Conditionern (Guar Quat oder Behentrimonium Chlorid) im Bereich 0,1 bis 0,2% erhöhen die Viskosität weiter.

Beispielrezepturen mit einer Gesamt-WAS (waschaktive Substanz) von nur ca. 6% aus Cocamidopropylbetain und Natrium Lauroylsarkosinat mit weiteren Cotensiden und Wirkstoffen ergeben hohe Viskositäten, zeigen zwar ähnlich wie die 6%igen Natrium Laurethsulfat/Cocamidopropylbetain Formulierungen eine gelartige Struktur und neigen dazu Fäden zu ziehen, haben aber immer noch ein deutlich besseres Anschäumen und angenehmes Hautgefühl. Hier erreicht der Formulierer die Grenzen der Formulierbarkeit mit geringen WAS-Gehalten, aber erstmals auch mit einem PEG- bzw. Natrium Laurethsulfat-freien System.

Eine Mischung aus Cocamidopropylbetain / Tegobetain C60 (2,5% WAS) und Natrium Myristoylsarkosinat (1,5% WAS) hat bei entsprechendem pH-Wert ebenfalls eine gelartige hohe Viskosität. Allerdings hat diese Mischung gewisse sensorische und auch ökonomische Nachteile.

Weitere Vorteile haben die erfindungsgemäßen Mischungen besonders in der geringeren Entfettung der Haut und den angenehmen konditionierenden Eigenschaften von Cocamidopropylbetain und Natrium Lauroylsarkosinat, was zu einem sehr gepflegtem Hautgefühl ohne einen zusätzlichen Wirkstoff führt. Dass die Reinigungsleistung im Vergleich zu Natrium Laurethsulfat-basierten Rezepturen mit theoretisch besseren Fettemulgierung etwas abfallen kann, hat in der täglichen Anwendung für den normalen Endverbraucher keine Relevanz.

Damit ist eine Mischung aus beispielsweise Cocamidopropylbetain und Natrium Lauroylsarkosinat bei einer benötigten Aktivmenge von rund 6 bis 12 % den marktüblichen Natrium Laurethsulfat und Cocamidopropylbetain Rezepturen mit 8 bis 16 % Aktivgehalt überlegen aufgrund besserer Sensorik und besserem Schaum. Durch den geringeren Aktivgehalt bei höherer Dosierung und dadurch, dass kein zusätzlicher Verdicker und Conditioner notwendig ist, wird ein etwaiger Preisnachteil der Wirkstoffe ausgeglichen.

Neben den genannten Vorteilen sind die erfindungsgemäßen Rezepturen auch farblos klar, was für bestimmte Marketingkonzepte vorteilhaft ist. Weiter löst die Formulierung übliche Conditioner wie Polyquaternium-10, Guar Hydroxypropyltrimonium Chlorid, Palmitamidopropyltrimonium Chlorid oder Behentrimonium Chlorid klar. Die resultierenden Mischungen haben einen starken konditionierenden Effekt. Dies kann sowohl für die Haarreinigung und -pflege als auch für die gewerbliche Hautreinigung interessant sein.

Derartige Rezepturen mit Mischungen von anionischen und kationischen Rohstoffen sind bislang im Rahmen der Natrium Laurethsulfat-haltigen Formulierungen unbekannt, da Natrium Laurethsulfat und ein kationisches Tensid normalerweise ein unlösliches Addukt bilden ("Präzipitat"), das aus der Lösung ausfällt.

Damit sind durch die erfindungsgemäße Formulierung Produkte darstellbar, die neu sind und neue, bislang unbekannte Eigenschaften und/oder Anwendungen zeigen.

Unerwarteter Weise zeigt die neue Formulierungsbasis neben den schon genannten Vorteilen eine hohe Kompatibilität mit allen eingesetzten Wirk- und Marketingrohstoffen der Kosmetik und verwendet breit eingesetzte und bekannte Tenside, woraus sich die besondere wirtschaftliche Bedeutung der Erfindung ergibt. Es bedarf keiner zeitaufwendigen toxikologischen Überprüfung der eingesetzten Rohstoffe, so dass dieser Kostenaspekt ebenfalls wegfällt.

Nachfolgend wird die vorliegende Erfindung anhand von weiteren Ausführungsbeispielen näher erläutert.
**Beispiel 1a:** Flüssigseife 5,6% WAS (kostengünstiges Produkt), klar, Viskosität ca. 1500 Mpas/40 s ALZ):

| | |
|---|---|
| Natrium Lauroylsarkosinat | 1,8% |
| Cocamidopropylbetain | 3,2% |
| Natrium Cocoamphoacetat | 0,6% |
| Polyquaternium-10 | 0,1% |
| Stearamidopropyl Dimethylamine | 0,5% |
| Milchsäure | 0,2% |
| Ameisensäure | ca. 0,2% |
| Zitronensäure/Kalilauge | pH adj. 4,5 bis 4,7 |
| Wasser, entkeimt, enthärtet und | |
| Farbe, Parfüm, Konservierung | ad 100 |

**Beispiel 1b:** Flüssigseife pH-neutral 8,5% WAS, klar, Viskosität ca. 3700 mPas/100 s ALZ:

| | |
|---|---|
| Natrium Cocoylglutamat | 3,5% |
| Lauraminoxid | 3,0% |
| Cocamidopropylbetain | 2,0% |
| Milchsäure | 0,1% |
| Ameisensäure | 0,3% |
| Zitronensäure/Kalilauge | pH adj. 6,15 - 6,35 |
| Wasser, entkeimt, enthärtet und | |
| Farbe, Parfüm, Konservierung | ad 100 |

**Beispiel 1c:** Milde Flüssigseife 8,6% WAS, klar mit verringertem Gehalt an Natrium Laureth Sulfate gegenüber handelsüblichen Marktprodukten, Viskosität ca. 3700 mPas/100 s ALZ:

| | |
|---|---|
| Natrium Lauroylsarkosinat | 2,0% |
| Cocamidopropylbetain | 3,8% |
| Sodium Laureth Sulfate | 2,8% |
| Milchsäure | 0,1% |
| Ameisensäure | 0,3% |
| Zitronensäure/Kalilauge | pH adj. 4,1 bis 4,4 |
| Wasser, entkeimt, enthärtet und | |
| Farbe, Parfüm, Konservierung | ad 100 |

**Beispiel 1d:** Flüssigseife 10,25% WAS, klar für Naturkosmetik-Konzept, Viskosität ca. 2700 mPas/84 s ALZ:

| | |
|---|---|
| Natrium Lauroylsarkosinat | 3,75% |
| Cocamidopropylbetain | 5,0% |
| Cocoamphoacetate | 1,5% |
| Milchsäure | 0,1% |
| Ameisensäure | 0,3% |
| Zitronensäure/Kalilauge | pH adj. 4,6 bis 4,8 |
| Wasser, entkeimt, enthärtet und | |
| Farbe, Parfüm, Konservierung | ad 100 |

**Beispiel 1e:** ph-neutrale Flüssigseife mit Alaninat 7,5% WAS, klar für Naturkosmetik-Konzept, Viskosität ca. 3700 mPas/100 s ALZ:

| | |
|---|---|
| Natrium Cocoylalaninat | 3,0% |
| Lauraminoxid | 4,5% |
| Milchsäure | 0,1% |
| Ameisensäure | 0,2% |
| Zitronensäure/Kalilauge | pH adj. 7,1 bis 7,4 |
| Wasser, entkeimt, enthärtet und | |
| Farbe, Parfüm, Konservierung | ad 100 |

**Beispiel 1f:** Flüssigseife 7,5% WAS (hochviskoses Produkt), klar, Viskosität ca. 5300 Mpas/224 s ALZ):

| | |
|---|---|
| Natrium Lauroylsarkosinat | 3,0% |
| Natrium Cocoamphoacetat | 4,5% |
| Stearamidopropyl Dimethylamine | 1,0% |
| Milchsäure | 0,2% |
| Ameisensäure | 0,4% |
| Zitronensäure/Kalilauge | (pH adj. 4,8 bis 5,1) |
| Wasser, entkeimt, enthärtet und | |
| Farbe, Parfüm, Konservierung | ad 100 |

**Beispiel 2:** Duschbad 8,8% WAS:

| | |
|---|---|
| Natrium Lauroylsarkosinat | 3% |
| Cocamidopropylbetain | 5% |
| Alkylpolyglycoside | 0,8% |
| Glycerylmonooleate | 0,3% |
| Panthenol | 0,5% |
| Glycerin | 1 % |
| Pflanzenextrakt | 0,1% |
| Conditioner | 0,1% |
| Parfüm | 0,3% |
| Farbstoffe | q. s. |
| Milchsäure | 0,2% |
| Ameisensäure | 0,3% |
| Zitronensäure/Kalilauge | (pH adj. 4,7 bis 5,0) |
| Wasser, entkeimt, enthärtet und | |
| Farbe, Parfüm, Konservierung | ad 100 |

**Beispiel 3a:** Shampoo 12% WAS:

| | |
|---|---|
| Natrium Lauroylsarkosinat | 4% |
| Cocamidopropylbetain | 6% |
| Natrium Cocoamphoacetat | 2% |
| Panthenol | 0,5% |
| Conditioner PQ-10 | 0,15% |
| Conditioner PQ-44 | 0,05% |
| Parfüm | 0,3% |
| Perlglanz,** | q. s. |
| Trübungsmittel | q. s. |
| Milchsäure | 0,2% |
| Ameisensäure | ca. 0,3% |
| Zitronensäure/Kalilauge | (pH adj. 4,8 bis 5,2) |
| Wasser, entkeimt, enthärtet und | |
| Farbe, Parfüm, Konservierung | ad 100 |
| **Glykoldistearate o. Homologe | |

**Beispiel 3b:** Mildes Shampoo 10,8% WAS mit verringertem Gehalt an Natrium Laureth Sulfate gegenüber handelsüblichen Marktprodukten:

| | |
|---|---|
| Natrium Lauroylsarkosinat | 3% |
| Cocamidopropylbetain | 5% |
| Sodium Laureth Sulfate | 2,8% |
| Panthenol | 0,5% |
| Guar-Quat | 0,15% |
| Conditioner PQ-44 | 0,05% |
| Parfüm | 0,3% |
| Perlglanz** | q. s. |
| Trübungsmittel | q. s. |
| Milchsäure | 0,2% |
| Ameisensäure | ca. 0,3% |
| Zitronensäure/Kalilauge | (pH adj. 4,8 bis 5,2) |
| Wasser, entkeimt, enthärtet und | |
| Farbe, Parfüm, Konservierung | ad 100 |
| **Glykoldistearate o. Homologe | |

**Beispiel 4a:** Shampoo mit Silicon 15% WAS:

| | |
|---|---|
| Natrium Lauroylsarkosinat | 5% |
| Cocamidopropylbetain | 8% |
| Cocamide MEA | 2% |
| Siliconöl 100- 1 Mio cs | 0,2% |
| Modifizierte Silicone | 0,5% |
| Silicon-Emulgator | 1% |
| Panthenol | 0,5% |
| Conditioner PQ-10 | 0,3% |
| Parfüm | 0,3% |
| Perlglanz** | q. s. |
| Trübungsmittel | q. s. |
| Milchsäure | 0,2% |
| Ameisensäure | 0,3% |
| Zitronensäure/Kalilauge | (pH adj. 4,0 bis 5,0) |
| Wasser, entkeimt, enthärtet und | |
| Farbe, Parfüm, Konservierung | ad 100 |
| **Glykoldistearate o. Homologe | |

**Beispiel 4b:** Mildes Silicon-Shampoo 18% WAS mit verringertem Gehalt an Natrium Laureth Sulfate gegenüber handelsüblichen Marktprodukten:

| | |
|---|---|
| Natrium Lauroylsarkosinat | 4% |
| Cocamidopropylbetain | 4% |
| Cocamide MEA | 2% |
| Sodium Laureth Sulfate | 8% |
| Siliconöl 100 - 1 Mio cs | 0,2% |
| Modifizierte Silicone | 0,5% |
| Silicon-Emulgator | 1% |
| Panthenol | 0,5% |
| Conditioner PQ-10 | 0,3% |
| Parfüm | 0,3% |
| Perlglanz,** | q. s. |
| Trübungsmittel | q. s. |
| Milchsäure | 0,2% |
| Ameisensäure | 0,3% |
| Zitronensäure/Kalilauge | (pH adj. 4,0 bis 5,0) |
| Wasser, entkeimt, enthärtet und | |
| Farbe, Parfüm, Konservierung | ad 100 |
| **Glykoldistearate o. Homologe | |

**Beispiel 5:** "Neues Conditioniershampoo 2 in 1" klar 10,3% WAS mit kationischem Tensid:

| | |
|---|---|
| Natrium Lauroylsarkosinat | 3% |
| Cocamidopropylbetain | 5% |
| Lauraminoxid | 2% |
| Natrium Cocoylglutamat | 0,3% |
| Panthenol | 0,5% |
| Conditioner PQ-10 | 0,15% |
| Behentrimoniumchlorid | 0,5% |
| Parfüm | 0,2% |
| Milchsäure | 0,2% |
| Ameisensäure | 0,3% |
| Zitronensäure/Kalilauge | (pH adj. 4,8 bis 5,5) |
| Wasser, entkeimt, enthärtet und | |
| Farbe, Parfüm, Konservierung | ad 100 |

### Vergleichsbeispiel 1

Um den erfindungsgemäß pH-Wert-abhängigen verdickenden Effekt spezifischer Aminosäuretenside demonstrieren zu können, wurden Vergleichsversuche angestellt, bei denen Rezepturen verwendet wurden, wie Sie im Stand der Technik genannt sind. Im Vergleichsbeispiel 1 wurde eine Tensidmischung verwendet, wie in Beispiel 1 der DE 197 23 763 A1 angegeben. Dabei wurden jedoch alle nichttensidischen Inhaltsstoffe weggelassen, die ggfs. mit zum Viskositätsaufbau beitragen können.

Die im Stand der Technik genannte Rezeptur wurde nachgestellt, wobei zunächst auf den dort angegebenen niedrigsten pH-Wert von 5,0 aus der Bandbreite 5,0 bis 8,5 eingestellt wurde. Wie in nachfolgender Tabelle zu sehen ist, zeigt die Rezeptur A, die wie in der vorliegenden Erfindung ein Aminosäuretensid enthielt, bei pH 5,0 eine Auslaufzeit von 13 s bedingt durch den hohen Tensidgehalt von 20,2% WAS. Die Rezeptur B ohne Natriumlauroylglutamat zeigt hingegen eine etwas höhere Auslaufzeit von 21 s trotz geringerem Aktivgehalt von 18,2% WAS. Beide Rezepturen haben bei pH 5,0 eine vergleichsweise geringe Viskosität von kleiner 1000 mPas, der Fachmann würde daher nach dem Stand der Technik Natriumchlorid zur Verdickung zugeben.

Die Rezeptur A mit Aminosäuretensid zeigt bei pH-Werten von 4,5 bzw. 3,9 einen drastischen Viskositätsaufbau, woraus ersichtlich ist, dass hier der erfindungsgemäße Effekt eintritt. Im Vergleich dazu zeigt Rezeptur B ohne Natriumlauroylglutamat hingegen nur einen geringen Viskositätsaufbau bei pH 4,5 bzw. 3,9. Die bei Rezeptur B beobachtete geringfügige kontinuierliche Viskositätssteigerung bei sinkendem pH-Wert resultiert vermutlich aus der zunehmenden Elektrolytkonzentration durch die zur pH-Werteinstellung verwendete Milchsäure. Die Zugabe von Natriumlauroylglutamat in Rezeptur A ist hingegen ursächlich für den deutlichen Viskositätsaufbau bei den angegebenen niedrigen pH-Werten um pH 3,9.

**Rezeptur nach DE 197 23 763 Seite 5 Beispiel 1 nur Tenside und Zitronensäure:**

| | A | B |
|---|---|---|
| Sodium Laureth Sulfate | 10,0 | 10,0 |
| C8-C22-Alkylglukosid | 5,0 | 5,0 |
| Natriumlauroylglutamat | 2,0 | ohne |
| Cocamidopropyl Betaine | 3,2 | 3,2 |
| Zitronensäure | 0,1 | 0,1 |
| Wasser | Ad 100 | ad 100 |
| | ALZ [s] | ALZ [s] |
| pH 5,0 (nach Vorschrift) | 13 | 21 |
| pH 4,5 | 162 | 30 |
| pH 3,9 | > 400 | 37 |
| pH 3,1 | 240 | 41 |
| Vergleich Viskositäten in mPas: | | |
| pH 3,9 | > 10000 mPas | ca. 1200 mPas |
| pH-Wert-Einstellung zur Einstellung pH 5,0 mit Zitronensäure 50%, unter pH 5,0 mit Milchsäure (80%) | | |

Das obige Beispiel zeigt im Vergleich zu binären Tensidmischungen bei dem verwendeten quaternären Tensidsystem einen breiteren Bereich ansteigender Viskosität, verursacht durch den hohen WAS-Gehalt von 20,2% und die Einflüsse der unterschiedlichen Tenside auf die Viskositätserhöhung mit Natriumlauroylglutamat (pH-Wert der jeweiligen binären Viskositätsmaxima).

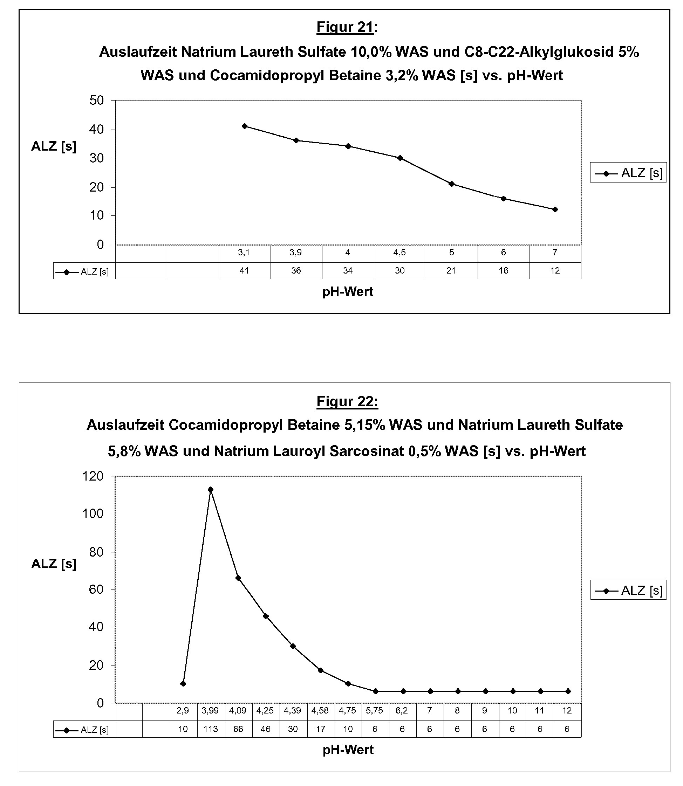

Der Viskositätsaufbau von Rezeptur A ist in Figur 20 dargestellt.
Figur 20 ist ein Diagramm, in dem die Auslaufzeit ALZ von Natrium Laureth Sulfate 10,0% WAS und C8-C22-Alkylglukosid 5% WAS und Natriumlauroylglutamate 2% WAS und Cocamidopropyl Betaine 3,2% WAS, in Sekunden abhängig vom pH-Wert dargestellt ist.
Der Viskositätsaufbau von Rezeptur B ist in Figur 21 dargestellt.
Figur 20 ist ein Diagramm, in dem die Auslaufzeit ALZ von Natrium Laureth Sulfate 10,0% WAS und C8-C22-Alkylglukosid 5% WAS und Cocamidopropyl Betaine 3,2% WAS, in Sekunden abhängig vom pH-Wert dargestellt ist.

### Vergleichsbeispiel 2

Exemplarisch wurde die Rezeptur in Spalte 16 Table 1 der US 5,866,110 nachgestellt und zunächst wurde auf die dort vorgegebenen pH-Werte eingestellt. Wiederum wurden, wie oben erläutert, alle nicht tensidischen Inhaltsstoffe, die ggfs. zur Viskositätssteigerung beitragen können, weggelassen. Wie in nachfolgender Tabelle zu sehen ist, zeigt die Rezeptur A mit Aminosäuretensid bei den in der US-Schrift angegebenen pH-Werten 6,2 und pH 5,75 keinen Viskositätsaufbau. Bei den pH-Werten von 4,5 bzw. 4,0 zeigt sich hingegen ein deutlicher Viskositätsaufbau, was den Effekt der vorliegenden Erfindung bestätigt. Die Vergleichs-Rezeptur B ohne Sodium Lauroyl Sarcosinate zeigt hingegen keinen erfindungsgemäßen Viskositätsaufbau bei pH 4,5 bzw. 4,0. Der bei Rezeptur B beobachtete kontinuierliche sehr geringe Viskositätsanstieg resultiert vermutlich, wie oben bereits in Vergleichsbeispiel 1 erwähnt, aus der zunehmenden Elektrolytkonzentration durch Milchsäure zur pH-Werteinstellung. Die Dosierung von Sodium Lauroyl Sarcosinate bei dem angegebenen spezifischen pH-Wert ist ursächlich für den Viskositätsaufbau.

Rezeptur nach US 5,866,110 Spalte 16 Table 1 nur Tenside und Zitronensäure:

| | A wie Patent | B |
|---|---|---|
| Cocamidopropyl Betaine | 5,15 | 5,15 |
| Sodium Laureth Sulfate | 5,8 | 5,8 |
| Sodium Lauroyl Sarcosinate | 0,5 | ohne |
| Zitronensäure | 0,1 | 0,1 |
| Wasser | Ad 100 | ad 100 |
| | | |
| | ALZ [s] | ALZ [s] |
| pH 6,2 (nach Vorschrift) | 6 | 6 |
| pH 5,75 (nach Vorschrift) | 6 | 6 |
| pH 4,5 | 23 | 7 |
| pH 4,0 | 113 | 9 |
| | | |
| Vergleich Viskositäten in mPas: | | |
| pH 4,0 | 4200 mPas | 1,5 mPas |
| pH-Wert-Einstellung mit Milchsäure (80%) | | |

Der Viskositätsaufbau von Rezeptur A wie Patent ist in Figur 22 dargestellt.

Figur 22 ist ein Diagramm, in dem die Auslaufzeit ALZ von Cocamidopropyl Betaine 5,15% WAS und Natrium Laureth Sulfate 5,8% WAS und Natrium Lauroyl Sarcosinat 0,5% WAS, in Sekunden abhängig vom pH-Wert dargestellt ist.
Der Viskositätsaufbau von Rezeptur B ist in Figur 27 dargestellt.
Figur 27 ist ein Diagramm, in dem die Auslaufzeit ALZ von Cocamidopropyl Betaine 5,15% WAS und Natrium Laureth Sulfate 5,8% WAS, in Sekunden abhängig vom pH-Wert dargestellt ist.

Dabei zeigt sich im Vergleich zu binären Tensidmischungen bei diesem ternären System ein breiterer Bereich ansteigender Viskosität, auch verursacht durch einen erhöhten WAS-Gehalt von 11,45%. Das Viskositätsmaximum von pH 4,0 liegt dabei deutlich verschieden gegenüber der Mischung von 3% WAS Natrium Cocoyl Sarcosinate mit 1,9% Cocamidopropyl Betaine und 1,4% Natrium Laureth Sulfate von pH 4,8 in Figur 17. Ein weiterer Beleg dafür, dass das Verhältnis der eingesetzten Tenside zueinander in ternären und komplexeren Mischungen auch die Lage des pH-Wertes des Viskositätsmaximums und dessen Verlauf beeinflusst. Der erfindungsgemäße Verdickungseffekt wird in diesem Beispiel schon von einer vergleichsweise geringen Menge Natrium Lauroyl Sarcosinat von 0,5% WAS ausgelöst.

## Patentansprüche

1. Flüssiges Reinigungs- und Pflegemittel für die Haar- und Körperreinigung mit guter Hautverträglichkeit, enthaltend wenigstens ein pH-sensitives Aminosäuretensid ausgewählt aus den Aminosäuretensiden Natriumlauroylsarkosinat, Natriumcocoylglutamat, Natriumcocoylalaninat und Natriumcocoylsarkosinat, Wasser, sowie mindestens einen pH-Wert-Regulator, der den pH-Wert des Gemischs auf einen spezifischen engen pH-Wert-Bereich von +/- 0,3 oder weniger bezogen auf einen pH-Wert einstellt, in dem das Gemisch ein absolutes Viskositätsmaximum zeigt oder eine relative Viskositätserhöhung von mindestens 100 % gegenüber einer Lösung dieses Aminosäuretensids in Wasser zeigt, nämlich auf einen pH-Wert von 4,65 bis 5,25 bei Verwendung von Natriumlauroylsarkosinat, von 4,3 bis 4,6 bei Verwendung von Natriumcocoylglutamat, von 5,4 bis 6, 0 bei Verwendung von Natriumcocoylalaninat, von 4,5 bis 5,0 bei Verwendung eines binären Tensidgemischs aus Natriumcocoylsarkosinat als pH-sensitives Aminosäuretensid sowie Cocamidopropylbetain, von 6,75 bis 7,35 bei Verwendung eines binären Gemischs aus Natriumcocoylsarkosinat als pH-sensitives Aminosäuretensid sowie einem Aminoxid oder auf einen pH-Wert von 5,9 bis 6,5 bei Verwendung eines ternären Tensidgemischs aus Natriumcocoylsarkosinat als pH-sensitives Aminosäuretensid, Cocamidopropylbetain sowie einem Aminoxid, wobei das Reinigungs- und Pflegemittel insgesamt nur 4 % bis 16 % an waschaktiven Substanzen enthält.

2. Reinigungs- und Pflegemittel nach Anspruch 1, **dadurch gekennzeichnet, dass** dieses in Form eines Duschbads, einer Flüssigseife, eines Shampoos oder eines Schaumbads vorliegt.

3. Reinigungs- und Pflegemittel nach Anspruch 1 oder 2, **dadurch gekennzeichnet, dass** dieses neben dem pH-sensitiven Aminosäuretensid weiterhin wenigstens ein weiteres nicht pH-sensitives anionisches Tensid und/oder wenigstens ein nichtionisches Tensid und/oder wenigstens ein kationisches und/oder ein amphoteres Tensid enthält.

4. Reinigungs- und Pflegemittel nach einem der Ansprüche 1 bis 3, **dadurch gekennzeichnet, dass** dieses insgesamt etwa 4 % bis etwa 16 % der genannten Tenside enthält, wobei es pH-sensitive Aminosäuretenside in einer Menge von etwa 1,8 Gew. % bis etwa 5 Gew. % enthält.

5. Reinigungs- und Pflegemittel nach einem der Ansprüche 1 bis 4, **dadurch gekennzeichnet, dass** dieses ein ternäres Tensidgemisch aus Natriumcocoylsarkosinat als pH-sensitives Aminosäuretensid, Cocamidopropylbetain sowie Lauraminoxid, enthält und dass mittels des pH-Wert-Regulators der pH-Wert auf einen Bereich von etwa 5,9 bis 6,5 (entspricht 6,2 +/- 0,3) eingestellt ist.

6. Reinigungs- und Pflegemittel nach einem der Ansprüche 1 bis 5, **dadurch gekennzeichnet, dass** als pH-Wert-Regulator mindestens eine organische Säure verwendet wird.

7. Reinigungs- und Pflegemittel nach einem der Ansprüche 1 bis 6, **dadurch gekennzeichnet, dass** dieses weitere nicht pH-sensitive anionische Tenside in einer Menge von bis zu etwa 5 Gew.% enthält und/oder nicht ionisches Tensid in einer Menge von bis zu etwa 5 Gew.% enthält und/oder kationisches Tensid in einer Menge von bis zu etwa 3 Gew.% enthält und/oder amphoteres Tensid in einer Menge von etwa 3 Gew.% bis etwa 8 Gew.% enthält.

8. Reinigungs- und Pflegemittel nach Anspruch 6, **dadurch gekennzeichnet, dass** als pH-Regulator Ameisensäure oder Milchsäure enthalten ist.

9. Reinigungs- und Pflegemittel nach einem der Ansprüche 1 bis 8, **dadurch gekennzeichnet, dass** dieses wenigstens ein pH-sensitives Aminosäuretensid gegebenenfalls im Gemisch mit weiteren Tensiden enthält und der pH-Wert des Gemischs mittels des pH-Wert-Regulators auf einen spezifischen engen pH-Wert-Bereich von +/- 0,3 oder weniger eingestellt ist, bezogen auf einen pH-Wert, in dem das Gemisch eine relative Viskositätserhöhung von mindestens 300 %, bevorzugt von mindestens 500 % gegenüber einem Gemisch gleicher Substanzen in Wasser zeigt, welches das oder die Aminosäuretenside nicht enthält.

10. Verfahren zur Herstellung eines Reinigungs- und Pflegemittels nach einem der Ansprüche 1 bis 9, **dadurch gekennzeichnet, dass** es mindestens einen Verfahrensschritt umfasst, in dem man einem Gemisch aus wenigstens einem pH-sensitiven Aminosäuretensid sowie gegebenenfalls weiteren nicht pH-sensitiven Tensiden und Wasser zur Einstellung der Viskosität mindestens einen pH-Wert-Regulator in einer solchen Menge zugibt, dass der pH-Wert des Gemischs auf einen spezifischen engen pH-Wert-Bereich von +/- 0,3 oder weniger bezogen auf einen pH-Wert eingestellt wird, in dem das Gemisch ein absolutes Viskositätsmaximum zeigt oder eine relative Viskositätserhöhung von mindestens 100 % gegenüber einer Lösung dieses Aminosäuretensids oder der Tenside in Wasser zeigt.

11. Verfahren zur Herstellung eines Reinigungs- und Pflegemittels nach Anspruch 10, **dadurch gekennzeichnet, dass** diesem bei der Herstellung Inhaltsstoffe nach Maßgabe eines oder mehrerer der Ansprüche 1 bis 9 zugegeben werden.

## Claims

1. A liquid cleansing and care product for hair and body cleansing having good compatibility with the skin, containing at least one pH-sensitive amino acid surfactant selected from the amino acid surfactants sodium lauroyl sarcosinate, sodium cocoyl glutamate, sodium cocoyl alaninate and sodium cocoyl sarcosinate, water, as well as at least one pH regulator which adjusts the pH of the mixture to a specifically narrow pH range of ± 0.3 or less with respect to a pH in which the mixture exhibits an absolute maximum of viscosity or a relative increase in viscosity of at least 100% compared with a solution of said amino acid surfactant in water, namely to a pH of 4.65 to 5.25 when using sodium lauroyl sarcosinate, of 4.3 to 4.6 when using sodium cocoyl glutamate, of 5.4 to 6.0 when using sodium cocoyl alaninate, of 4.5 to 5.0 when using a binary surfactant mixture of sodium cocoyl sarcosinate as the pH-sensitive amino acid surfactant as well as cocamidopropyl betaine, of 6.75 to 7.35 when using a binary mixture of sodium cocoyl sarcosinate as the pH-sensitive amino acid surfactant as well as an amine oxide, or to a pH of 5.9 to 6.5 when using a ternary surfactant mixture of sodium cocoyl sarcosinate as the pH-sensitive amino acid surfactant, cocamidopropyl betaine as well as an amine oxide, wherein the cleansing and care product contains only 4% to 16% of detergent substances in total.

2. The cleansing and care product as claimed in claim 1, **characterized in that** it is in the form of a shower product, a liquid soap, a shampoo or a foam bath.

3. The cleansing and care product as claimed in claim 1 or claim 2, **characterized in that** in addition to the pH-sensitive amino acid surfactant, it further contains at least one further non-pH-sensitive anionic surfactant and/or at least one non-ionic surfactant and/or at least one cationic and/or amphoteric surfactant.

4. The cleansing and care product as claimed in one of claims 1 to 3, **characterized in that** it contains a total of approximately 4% to approximately 16% of said surfactants, wherein it contains pH-sensitive amino acid surfactants in a quantity of approximately 1.8% by weight to approximately 5% by weight.

5. The cleansing and care product as claimed in one of claims 1 to 4, **characterized in that** it contains a ternary surfactant mixture of sodium cocoyl sarcosinate as the pH-sensitive amino acid surfactant, cocamidopropyl betaine as well as lauramine oxide, and **in that** the pH is adjusted to a value in the range from approximately 5.9 to 6.5 (corresponding to 6.2 ± 0.3) using the pH regulator.

6. The cleansing and care product as claimed in one of claims 1 to 5, **characterized in that** at least one organic acid is used as the pH regulator.

7. The cleansing and care product as claimed in one of claims 1 to 6, **characterized in that** it further contains non-pH-sensitive anionic surfactants in a quantity of up to approximately 5% by weight and/or non-ionic surfactant in a quantity of up to approximately 5% by weight and/or cationic surfactant in a quantity of up to approximately 3% by weight and/or amphoteric surfactant in a quantity of approximately 3% by weight to approximately 8% by weight.

8. The cleansing and care product as claimed in claim 6, **characterized in that** it contains formic acid or lactic acid as the pH regulator.

9. The cleansing and care product as claimed in one of claims 1 to 8, **characterized in that** it contains at least one pH-sensitive amino acid surfactant, optionally as a mixture with further surfactants, and the pH of the mixture is adjusted using the pH regulator to a specifically narrow pH range of ± 0.3 or less with respect to a pH in which the mixture has a relative increase in viscosity of at least 300%, preferably of at least 500% with respect to a mixture of the same substances in water which does not contain the amino acid surfactant or surfactants.

10. A method for the manufacture of a cleansing and care product as claimed in one of claims 1 to 9, **characterized in that** it comprises at least one step of the method in which, in order to adjust the viscosity, at least one pH regulator is added to a mixture of at least one pH-sensitive amino acid surfactant as well as optional further non-pH-sensitive surfactants and water, in a quantity such that the pH of the mixture is adjusted to a specifically narrow pH range of ± 0.3 or less with respect to a pH in which the mixture exhibits an absolute maximum of viscosity or a relative increase in viscosity of at least 100% compared with a solution of said amino acid surfactant or the surfactants in water.

11. The method for the manufacture of a cleansing and care product as claimed in claim 10, **characterized in that** ingredients in accordance with one or more of claims 1 to 9 are added to it.

## Revendications

1. Produit de nettoyage et d'entretien liquide pour le nettoyage des cheveux et du corps avec une bonne tolérance cutanée, contenant au moins un tensioactif à base d'acides aminés sensible au pH sélectionné à partir des tensioactifs à base d'acides aminés :
lauroyl sarconsinate de sodium, cocoyl glutamate de sodium, cocoyl alaninate de sodium et cocoyl sarcosinate de sodium, eau, ainsi qu'au moins un régulateur du pH, qui régule la valeur du pH du mélange sur une plage de valeurs de pH spécifique étroite de +/- 0,3 ou moins en se référant à une valeur de pH, dans lequel le mélange indique un maximum absolu de viscosité ou une augmentation de la viscosité relative d' au moins 100 % par rapport à une solution de ce tensioactif à base d'acides aminés dans l'eau, notamment à une valeur de pH de 4,65 à 5,25 lors de l'emploi de lauroyl sarcosinate de sodium, de 4,3 à 4,6 lors de l'emploi du cocoyl glutamate de sodium, de 5,4 à 6,0 lors de l'emploi de cocoyl sarcosinate de sodium, de 4,5 à 5,0 lors de l'emploi d'un mélange binaire de tensioactifs à partir de cocoyl sarconsinate de sodium en tant que tensioactif à base d'acides aminés sensible au pH ainsi que de cocamidopropylbétaine, de 6,75 à 7,35 lors de l'emploi d'un mélange binaire de cocoyl sarconsinate de sodium en tant qu'agent tensioactif à base d'acides aminés ainsi qu'un oxyde aminé ou sur une valeur de pH de 5,9 à 6,5 lors de l'emploi d'un mélange de tensioactifs à base d'acides aminés sensible au pH, de cocoyl sarcosinate de sodium en tant que tensioactif à base d'acides aminés sensible au pH, de cocamidopropylbétaine ainsi qu'un oxyde aminé, le produit de nettoyage et d'entretien ne contenant au total que 4% à 16% de substances détergentes.

2. Produit de nettoyage et d'entretien selon la revendication 1, **caractérisé en ce que** celui-ci existe sous la forme d'un produit de bain douche, d'un savon liquide, d'un shampoing ou d'un produit de bain moussant.

3. Produit de nettoyage et d'entretien selon la revendication 1 ou 2, **caractérisé en ce que** celui-ci contient en plus du tensioactif à base d'acides aminés sensible au pH, de plus, au moins un autre tensioactif anionique non sensible au pH et/ou au moins un tensioactif non ionique et/ou au moins un tensioactif cationique et/ou amphotère.

4. Produit de nettoyage et d'entretien selon l'une quelconque des revendications 1 à 3, **caractérisé en ce que** celui-ci contient au total environ 4% à environ 16% des tensioactifs cités, celui-ci contenant des tensioactifs à base d'acides aminés sensibles au pH dans une autre quantité d'environ 1,8 % à environ 5 %/poids.

5. Produit de nettoyage et d'entretien selon l'une quelconque des revendications 1 à 4, **caractérisé en ce que** celui-ci contient un mélange ternaire d'agents tensioactifs de cocoyl sarcosinate de sodium en tant qu'agent tensioactif à base d'acides aminés sensible au pH, de cocamidopropylbétaine ainsi que de lauraminoxyde et **en ce que** la valeur de pH est réglée au moyen du régulateur de valeur de pH sur une plage d'environ 5,9 à 6,5 (correspondant à 6,2 +/- 0,3).

6. Produit de nettoyage et d'entretien selon l'une quelconque des revendications 1 à 5, **caractérisé en ce qu'**au moins un acide organique est utilisé comme régulateur de la valeur de pH.

7. Produit de nettoyage et d'entretien selon l'une quelconque des revendications 1 à 6, **caractérisé en ce que** celui-ci contient d'autres tensioactifs anioniques sensibles au pH dans une quantité allant jusqu'à environ 5 %/poids et/ou un tensioactif non ionique dans une quantité allant jusqu'à environ 5 %/poids et/ou un tensioactif cationique dans une quantité allant jusqu'à environ 3 %/poids et/ou un tensioactif amphotère dans une quantité d'environ 3 %/poids jusqu'à environ 8 %/poids.

8. Produit de nettoyage et d'entretien selon la revendication 6, **caractérisé en ce que** de l'acide formique ou de l'acide lactique est contenu en tant que régulateur de la valeur de pH.

9. Produit de nettoyage et d'entretien selon l'une quelconque des revendications 1 à 8, **caractérisé en ce que** celui contient au moins un tensioactif à base d'acides aminés sensible au pH le cas échéant dans un mélange avec d'autres tensioactifs et la valeur du pH du mélange est réglée au moyen du régulateur de valeur de pH sur une plage de valeurs de pH spécifique étroite de +/- 0,3 ou moins, en se référant à une valeur de pH, dans lequel le mélange indique une augmentation de viscosité relative d'au moins 300 %, de préférence d'au moins 500 %, par rapport à un mélange de substances analogues dans l'eau, lequel ne contient pas le ou les tensioactifs à base d'acides aminés.

10. Procédé destiné à fabriquer un produit de nettoyage et d'entretien selon l'une quelconque des revendications 1 à 9, **caractérisé en ce qu'**il comprend au moins une étape de procédé dans laquelle on ajoute à un mélange d'au moins un tensioactif à base d'acides aminés sensible au pH ainsi que le cas échéant d'autres tensioactifs non sensibles au PH et d'eau pour ajuster la viscosité, au moins un régulateur de valeur de pH dans une quantité telle que la valeur de pH du mélange est réglée sur une plage de valeurs de pH spécifique étroite de +/- 0,3 ou moins en se référant à une valeur de pH, dans lequel le mélange indique un maximum de viscosité absolu ou indique une augmentation de la viscosité relative d'au moins 100 % par rapport à une solution de ce tensioactif à base d'acides aminés ou des tensioactifs dans l'eau.

11. Procédé destiné à fabriquer un produit de nettoyage et d'entretien selon la revendication 10, **caractérisé en ce qu'**on ajoute à celui-ci, lors de la fabrication, des ingrédients conformément à l'une quelconque ou à plusieurs des revendications 1 à 9.
